# EUROPEAN PATENT APPLICATION

(11) **EP 4 516 315 A1**
(43) Date of publication of application: **05.03.2025**
(21) Application number: 23795536.4
(22) Date of filing: 27.04.2023
(51) Int. Cl.: A61K 39/395, C07K 16/28, A61P 37/02, A61P 11/00, A61P 29/00

(54) **ANTI-HUMAN IL-4RA ANTIBODY AND APPLICATION THEREOF**

(30) Priority: 29.04.2022 CN 202210473874
(71) Applicant: Akeso Biopharma, Inc., Zhongshan, Guangdong 528437 (CN)
(72) Inventor: XIA, Yu, Zhongshan, Guangdong 528437 (CN); WANG, Zhongmin, Zhongshan, Guangdong 528437 (CN); LI, Baiyong, Zhongshan, Guangdong 528437 (CN)
(74) Representative: Altmann Stößel Dick Patentanwälte PartG mbB
(86) International application number: PCT/CN2023/091111
(87) International publication number: WO 2023/208104

(57) **Abstract**

The present invention relates to an anti-human interleukin-4 receptor A antibody, a pharmaceutical composition thereof or a kit, and an application thereof in the treatment of eosinophilic esophagitis.

## Description

### TECHNICAL FIELD

The present invention relates to the field of allergic disease treatment and molecular immunology, and particularly, to an anti-human interleukin-4-receptor A antibody, a pharmaceutical composition or a kit comprising the same, and use thereof, for example, in treating eosinophilic esophagitis.

### BACKGROUND

Interleukin-4 receptor (IL-4R) is a transmembrane receptor that exists in two distinct forms: type I IL-4R, which consists of an IL-4R α subunit (referred to as IL-4RA herein) with high affinity and a γ_{c} subunit with moderate affinity, and binds to interleukin-4 (IL-4) and mediates the biological functions of cell proliferation, activation, and the like induced by IL-4; and type II IL-4R, which consists of an IL-4R α subunit (IL-4RA) with high affinity and an interleukin-13 receptor α subunit (IL-13Rα), and is a homologous functional receptor for interleukin-13 (IL-13) capable of binding to IL-13 (Wang and Secombes, Cytokine, 2015, 75(1):8-13). IL-4R is expressed in T cells, B cells, hematopoietic stem cells and endothelial cells, epithelial cells, muscles, fibroblasts, hepatocytes, and brain tissues. IL-4RA, along with γ_{c} subunit or IL-13R α subunit, activates various non-receptor protein tyrosine kinases in cytoplasm after IL-4 binds to its receptor, further initiating downstream signal transduction pathways (Nelms et al., Annu. Rev. Immunol., 1999, 17:701-738; LaPorte et al., Cell, 2008, 132(2):259-272).

IL-4RA can bind to IL-4 and IL-13 with high affinity, and is a major functional subunit of the foregoing type I and type II IL-4R. The inhibition of IL-4RA can effectively block relevant biological functions mediated by IL-4 and IL-13 (Gessner et al., Immunobiology, 2000, 201:285).

Eosinophilic esophagitis (EoE) is a chronic esophageal disease caused by eosinophilic infiltration into the esophagus, which is prevalent among adolescents and children, and presents with symptoms such as heartburn/regurgitation, vomiting, dysphagia, food impaction, and even abdominal pain (Gonsalves Nirmala P, Aceves Seema S, Diagnosis and treatment of eosinophilic esophagitis [J]. J Allergy Clin Immunol, 2020, 145:1-7). Dietary restrictions and in some cases, repeated hospital interventions are required for EoE patients, leading to a low quality of life for the patients.

EoE is often associated with allergic diseases, and 50%-60% of EoE patients have a history of allergies, such as rhinitis, bronchial asthma, atopic dermatitis and other atopic diseases, along with corresponding symptoms. Gastroesophageal reflux disease (GERD), allergens (primarily food allergens), alterations in the epithelial barrier and possible microbiota binding may trigger EoE. Food allergens and other factors penetrate the epithelium and activate receptors and inflammatory cells such as eosinophils, which secrete cytokines, inducing chronic inflammation, tissue damage, and fibrosis (Vinit C, Dieme A, Courbage S, et al., Eosinophilic esophagitis: Pathophysiology, diagnosis, and management [J]. Archives De Pédiatrie, 2019, 26(3):182-190).

EoE is an immune inflammatory response mediated by helper T cell 2 (Th2). IL-4 is able to induce the differentiation of naive T cells into Th2 cells, and Th2 cells secrete IL-5, which enhances eosinophil recruitment by promoting eotaxin release and eosinophil survival, exacerbating the inflammatory response (Joshua B.Wechsler, Ikuo Hirano, Bai Xiaoyin, Qian Jiaming, Biological therapies for eosinophilic gastrointestinal diseases [J]. Chinese Journal of Allergy & Clinical Immunology, 2018, 12(04):437-444). The second clinical Phase 3 (Part B) trial of dupilumab, an anti-human IL-4RA monoclonal antibody, evaluated the study use of dupilumab in eosinophilic esophagitis patients aged 12 and older. The results showed a significant improvement in clinical and histological disease indices in patients taking 300 mg of dupilumab weekly compared to those in the placebo group, with a 64% reduction in EoE symptoms compared to 41% in the placebo group.

### SUMMARY

After intensive studies and creative efforts, the inventors used mammalian cell expression systems to express recombinant human IL-4RA as an antigen to immunize mice, and obtained hybridoma cells by fusion of mouse spleen cells and myeloma cells. The inventors obtained the following hybridoma cell lines by screening a large number of the samples:
The inventors found that:
the hybridoma cell line 13E5 is capable of secreting and producing a specific antibody (named as 13E5) that specifically binds to the human IL-4RA, and the antibody is capable of blocking the binding of human IL-4RA to IL-4 effectively. Furthermore, the inventors have creatively prepared humanized anti-human IL-4RA antibodies (named as 13E5H1L1, 13E5H2L2, 13E5H3L3, 13E5H4L4, and 13E5H4L2, respectively).

The foregoing antibodies can effectively bind to the human IL-4RA, block the binding of human IL-4RA to a ligand IL-4 or IL-13 thereof, and inhibit the activation of the downstream signaling pathway of the human IL-4RA. The antibodies have potential in preparing a medicament for preventing and treating eosinophilic esophagitis.

The present invention is detailed below.
1. Provided is an antibody or an antigen-binding fragment thereof, particularly, the antibody or the antigen-binding fragment thereof binding to IL-4RA, preferably human IL-4RA, for use in treating eosinophilic esophagitis, wherein: according to the Kabat, IMGT, Chothia, or AbM numbering system, the antibody comprises:
   an HCDR1, an HCDR2, and an HCDR3 comprised in a heavy chain variable region set forth in SEQ ID NO: 2, and an LCDR1, an LCDR2, and an LCDR3 comprised in a light chain variable region set forth in SEQ ID NO: 4;
   preferably, according to the IMGT numbering system, the antibody comprises:
      an HCDR1 comprising or consisting of a sequence set forth in SEQ ID NO: 5 or a variant thereof,
      an HCDR2 comprising or consisting of a sequence set forth in SEQ ID NO: 6 or a variant thereof, and
      an HCDR3 comprising or consisting of a sequence set forth in SEQ ID NO: 7 or a variant thereof; and
      the antibody further comprises:
         an LCDR1 comprising or consisting of a sequence set forth in SEQ ID NO: 8 or a variant thereof,
         an LCDR2 comprising or consisting of a sequence set forth in SEQ ID NO: 9 or a variant thereof, and
         an LCDR3 comprising or consisting of a sequence set forth in SEQ ID NO: 10 or a variant thereof,
         wherein a sequence of the variant is a sequence having at least 90%, preferably at least 91%, 92%, 93%, 94%, 95%, 96%, 97%, 98%, or 99% sequence identity to the corresponding sequence.
2. Provided is the antibody according to item 1, wherein the antibody comprises:
   (1)
      (i) a heavy chain variable region comprising or consisting of:
         an amino acid sequence set forth in SEQ ID NO: 2 or a variant thereof, and
      (ii) a light chain variable region comprising or consisting of:
         an amino acid sequence set forth in SEQ ID NO: 4 or a variant thereof;
   (2)
      (i) a heavy chain variable region comprising or consisting of:
         an amino acid sequence set forth in SEQ ID NO: 12 or a variant thereof, and
      (ii) a light chain variable region comprising or consisting of:
         an amino acid sequence set forth in SEQ ID NO: 14 or a variant thereof;
   (3)
      (i) a heavy chain variable region comprising or consisting of:
         an amino acid sequence set forth in SEQ ID NO: 16 or a variant thereof, and
      (ii) a light chain variable region comprising or consisting of:
         an amino acid sequence set forth in SEQ ID NO: 18 or a variant thereof;
   (4)
      (i) a heavy chain variable region comprising or consisting of:
         an amino acid sequence set forth in SEQ ID NO: 20 or a variant thereof, and
      (ii) a light chain variable region comprising or consisting of:
         an amino acid sequence set forth in SEQ ID NO: 22 or a variant thereof;
   (5)
      (i) a heavy chain variable region comprising or consisting of:
         an amino acid sequence set forth in SEQ ID NO: 24 or a variant thereof, and
      (ii) a light chain variable region comprising or consisting of:
         an amino acid sequence set forth in SEQ ID NO: 26 or a variant thereof;
   (6)
      (i) a heavy chain variable region comprising or consisting of:
         an amino acid sequence set forth in SEQ ID NO: 24 or a variant thereof, and
      (ii) a light chain variable region comprising or consisting of:
         an amino acid sequence set forth in SEQ ID NO: 18 or a variant thereof,
         alternatively wherein a sequence of the variant is a sequence having at least 80%, 85%, or 90%, preferably at least 91%, 92%, 93%, 94%, 95%, 96%, 97%, 98%, or 99% sequence identity to the corresponding sequence.
3. Provided is the antibody or the antigen-binding fragment thereof according to any one of items 1 and 2, wherein the antibody further comprises framework regions FR-H1, FR-H2, FR-H3, and FR-H4 in the heavy chain variable region, and framework regions FR-L1, FR-L2, FR-L3, and FR-L4 in the light chain variable region, wherein
   (1) the FR-H1 comprises or consists of the amino acid sequence of SEQ ID NO: 27 or a variant thereof;
      the FR-H2 comprises or consists of the amino acid sequence of SEQ ID NO: 28 or a variant thereof;
      the FR-H3 comprises or consists of the amino acid sequence of SEQ ID NO: 29 or a variant thereof;
      the FR-H4 comprises or consists of the amino acid sequence of SEQ ID NO: 30 or a variant thereof;
      the FR-L1 comprises or consists of the amino acid sequence of SEQ ID NO: 31 or a variant thereof;
      the FR-L2 comprises or consists of the amino acid sequence of SEQ ID NO: 32 or a variant thereof;
      the FR-L3 comprises or consists of the amino acid sequence of SEQ ID NO: 33 or a variant thereof; and
      the FR-L4 comprises or consists of the amino acid sequence of SEQ ID NO: 34 or a variant thereof;
   (2) the FR-H1 comprises or consists of the amino acid sequence of SEQ ID NO: 35 or a variant thereof;
      the FR-H2 comprises or consists of the amino acid sequence of SEQ ID NO: 36 or a variant thereof;
      the FR-H3 comprises or consists of the amino acid sequence of SEQ ID NO: 37 or a variant thereof;
      the FR-H4 comprises or consists of the amino acid sequence of SEQ ID NO: 38 or a variant thereof;
      the FR-L1 comprises or consists of the amino acid sequence of SEQ ID NO: 39 or a variant thereof;
      the FR-L2 comprises or consists of the amino acid sequence of SEQ ID NO: 40 or a variant thereof;
      the FR-L3 comprises or consists of the amino acid sequence of SEQ ID NO: 41 or a variant thereof; and
      the FR-L4 comprises or consists of the amino acid sequence of SEQ ID NO: 42 or a variant thereof;
   (3) the FR-H1 comprises or consists of the amino acid sequence of SEQ ID NO: 43 or a variant thereof;
      the FR-H2 comprises or consists of the amino acid sequence of SEQ ID NO: 44 or a variant thereof;
      the FR-H3 comprises or consists of the amino acid sequence of SEQ ID NO: 45 or a variant thereof;
      the FR-H4 comprises or consists of the amino acid sequence of SEQ ID NO: 46 or a variant thereof;
      the FR-L1 comprises or consists of the amino acid sequence of SEQ ID NO: 47 or a variant thereof;
      the FR-L2 comprises or consists of the amino acid sequence of SEQ ID NO: 48 or a variant thereof;
      the FR-L3 comprises or consists of the amino acid sequence of SEQ ID NO: 49 or a variant thereof; and
      the FR-L4 comprises or consists of the amino acid sequence of SEQ ID NO: 50 or a variant thereof;
   (4) the FR-H1 comprises or consists of the amino acid sequence of SEQ ID NO: 51 or a variant thereof;
      the FR-H2 comprises or consists of the amino acid sequence of SEQ ID NO: 52 or a variant thereof;
      the FR-H3 comprises or consists of the amino acid sequence of SEQ ID NO: 53 or a variant thereof;
      the FR-H4 comprises or consists of the amino acid sequence of SEQ ID NO: 54 or a variant thereof;
      the FR-L1 comprises or consists of the amino acid sequence of SEQ ID NO: 55 or a variant thereof;
      the FR-L2 comprises or consists of the amino acid sequence of SEQ ID NO: 56 or a variant thereof;
      the FR-L3 comprises or consists of the amino acid sequence of SEQ ID NO: 57 or a variant thereof; and
      the FR-L4 comprises or consists of the amino acid sequence of SEQ ID NO: 58 or a variant thereof;
   (5) the FR-H1 comprises or consists of the amino acid sequence of SEQ ID NO: 59 or a variant thereof;
      the FR-H2 comprises or consists of the amino acid sequence of SEQ ID NO: 60 or a variant thereof;
      the FR-H3 comprises or consists of the amino acid sequence of SEQ ID NO: 61 or a variant thereof;
      the FR-H4 comprises or consists of the amino acid sequence of SEQ ID NO: 62 or a variant thereof;
      the FR-L1 comprises or consists of the amino acid sequence of SEQ ID NO: 63 or a variant thereof;
      the FR-L2 comprises or consists of the amino acid sequence of SEQ ID NO: 64 or a variant thereof;
      the FR-L3 comprises or consists of the amino acid sequence of SEQ ID NO: 65 or a variant thereof; and
      the FR-L4 comprises or consists of the amino acid sequence of SEQ ID NO: 66 or a variant thereof,
      wherein a sequence of the variant has at least 80%, 85%, or 90%, preferably at least 91%, 92%, 93%, 94%, 95%, 96%, 97%, 98%, or 99% sequence identity to the corresponding sequence.
4. Provided is the antibody or the antigen-binding fragment thereof according to any one of items 1-3, wherein the antibody is a humanized antibody, a chimeric antibody, or a multispecific antibody (e.g., bispecific antibody).
5. Provided is the antibody or the antigen-binding fragment thereof according to item 4, wherein the constant region of the antibody is humanized, preferably derived from human IgG, more preferably IgG4.
6. Provided is the antibody or the antigen-binding fragment thereof according to item 5, wherein the heavy chain constant region of the antibody is an Ig gamma-4 chain C region, more preferably the Ig gamma-4 chain C region of GenBank ACCESSION No: P01861.1; the light chain constant region is an Ig kappa chain C region, more preferably the Ig kappa chain C region of GenBank ACCESSION No: P01834.
7. Provided is the antibody or the antigen-binding fragment thereof according to any one of items 1-6, wherein the antigen-binding fragment is selected from Fab, Fab', F(ab')₂, Fd, Fv, dAb, Fab/c, a complementarity determining region (CDR) fragment, a single chain antibody (e.g., scFv), a bivalent antibody, or a domain antibody.
8. Provided is a biomaterial, wherein the biomaterial is selected from an antibody conjugate, a multispecific antibody (preferably a bispecific antibody), a fusion protein, or a pharmaceutical composition for use in treating eosinophilic esophagitis, and the biomaterial comprises the antibody or the antigen-binding fragment thereof according to any one of items 1-7,
   wherein preferably, the antibody conjugate further comprises a conjugated moiety coupled to the antibody or the antigen-binding fragment thereof, the conjugated moiety being selected from a purification tag (e.g., a His tag), a cytotoxic agent, a detectable label, a radioisotope, a luminescent substance, a colored substance, an enzyme, or polyethylene glycol;
   preferably, the multispecific antibody further comprises an antibody or an antigen-binding fragment against another antigen and/or another antigenic epitope;
   preferably, the pharmaceutical composition further comprises a pharmaceutically acceptable carrier and/or excipient.
   Preferably, the pharmaceutical composition is used alone or in combination with one or more medicaments.
   Preferably, the pharmaceutical composition is in a dosage form suitable for oral administration to the gastrointestinal (GI) tract, preferably the dosage form being selected from tablets, capsules, pills, powders, granules, emulsions, microemulsions, solutions, suspensions, syrups, and elixirs; or the pharmaceutical composition is in a dosage form suitable for subcutaneous injection, intradermal injection, intravenous injection, intramuscular injection, or intralesional injection.
9. Provided is use of the antibody or the antigen-binding fragment thereof according to any one of items 1-7 and the biomaterial according to item 8 in preparing a medicament or kit for treating eosinophilic esophagitis, wherein preferably, the kit further comprises an additional therapeutic agent selected from one or more of the following: an IL-4/IL-13 pathway inhibitor, an IL-1β inhibitor, an IL-5 inhibitor, an IL-8 inhibitor, an IL-9 inhibitor, an IL-13 inhibitor, an IL-17 inhibitor, an IL-25 inhibitor, a JAK inhibitor, a STAT6 inhibitor, a TNF inhibitor, an eotaxin-3 inhibitor, an IgE inhibitor, a prostaglandin D2 inhibitor, an immunosuppressive agent, a corticosteroid, a glucocorticoid, a long-acting β2-agonist (e.g., salmeterol or formoterol), a proton pump inhibitor, a decongestant, an antihistamine and a non-steroidal anti-inflammatory agent (NSAID), an NSAID (non-steroidal anti-inflammatory drug), an antibiotic, an antibacterial agent, an antiviral agent, an antifungal agent, and a drug for treating tumors (preferably a chemotherapeutic agent or a growth inhibitor, a targeted therapeutic agent, an antibody-drug conjugate, a T cell expressing chimeric antigen receptors, an antibody or an antigen-binding fragment thereof, an angiogenesis inhibitor, an anti-tumor agent, a cancer vaccine, an adjuvant and a combination thereof, an alkylating agent, an antimetabolite, an antibiotic, a botanical drug, and/or a hormonal drug),
   wherein the IL-4/IL-13 pathway inhibitor is not the antibody or the antigen-binding fragment thereof according to any one of items 1-7, and
   preferably, the IL-4/IL-13 pathway inhibitor is selected from an anti-IL-4 antibody, an anti-IL-13 antibody, an anti-IL-4/IL-13 antibody, an IL-4 receptor (IL-4R) inhibitor (e.g., a dupilumab antibody or a biological equivalent thereof, AMG317, or MEDI9314), a JAK inhibitor, or a STAT6 inhibitor.
10. Provided is a method for treating eosinophilic esophagitis, comprising administering to a subject in need the antibody or the antigen-binding fragment thereof according to any of items 1-7 and the biomaterial according to item 8, wherein preferably, the subject is selected from a mammal (e.g., a rodent, a feline, a canine, and a primate), and preferably the subject is a human, more preferably an infant aged 0-1, a child aged 1-5, a juvenile aged 6-12, an adolescent aged 12-18, and an adult aged above 18.
11. Provided is the method according to item 10, further comprising administering to the subject an additional therapeutic agent or a combination therapy, wherein the therapeutic agent is selected from one or more of the following: an IL-4/IL-13 pathway inhibitor, an IL-1β inhibitor, an IL-5 inhibitor, an IL-8 inhibitor, an IL-9 inhibitor, an IL-13 inhibitor, an IL-17 inhibitor, an IL-25 inhibitor, a JAK inhibitor, a STAT6 inhibitor, a TNF inhibitor, an eotaxin-3 (CCL26) inhibitor, an IgE inhibitor, a prostaglandin D2 inhibitor, an immunosuppressive agent, a corticosteroid, a glucocorticoid, a long-acting β2-agonist (e.g., salmeterol or formoterol), a proton pump inhibitor, a decongestant, an antihistamine and a non-steroidal anti-inflammatory agent (NSAID), an NSAID (non-steroidal anti-inflammatory drug), an antibiotic, an antibacterial agent, an antiviral agent, an antifungal agent, a drug for treating tumors (preferably a chemotherapeutic agent or a growth inhibitor, a targeted therapeutic agent, an antibody-drug conjugate, a T cell expressing chimeric antigen receptors, an antibody or an antigen-binding fragment thereof, an angiogenesis inhibitor, an anti-tumor agent, a cancer vaccine, an adjuvant and a combination thereof, an alkylating agent, an antimetabolite, an antibiotic, a botanical drug, and/or a hormonal drug), or a combination thereof, wherein the combination therapy comprises diet therapy and bougienage of oesophagus; the IL-4/IL-13 pathway inhibitor is not the antibody or the antigen-binding fragment thereof according to any one of items 1-7;
   preferably, the IL-4/IL-13 pathway inhibitor is selected from an anti-IL-4 antibody, an anti-IL-13 antibody, an anti-IL-4/IL-13 antibody, an IL-4 receptor (IL-4R) inhibitor (e.g., a dupilumab antibody or a biological equivalent thereof, AMG317, or MEDI9314), a JAK inhibitor, or a STAT6 inhibitor;
   more preferably, the additional therapeutic agent is administered simultaneously or sequentially with the antibody or the antigen-binding fragment thereof according to any one of items 1-7.
12. Provided is a hybridoma cell line having an accession number of CCTCC NO: C2018131 or a monoclonal antibody secreted by the hybridoma cell line for use in treating eosinophilic esophagitis.

In certain embodiments of the present invention, provided is use of an IL-4/IL-13 pathway inhibitor in preparing a medicament for treating, inhibiting, or preventing eosinophilic esophagitis in an individual.

In certain embodiments of the present invention, provided is use of an antibody or an antigen-binding fragment thereof binding to IL-4RA in preparing a medicament for treating, inhibiting, or preventing eosinophilic esophagitis in an individual.

In the present invention, the treatment of EoE comprises alleviating at least one symptom or sign including, but not limited to, eosinophilic infiltration of the esophagus, thickening of the esophageal wall, esophagitis, the presence of an intraesophageal tubular ring or protrusion, thoracoabdominal pain, food refusal, vomiting, dysphagia, and food impaction.

In some embodiments of the present invention, the "eosinophilic infiltration" refers to the presence of eosinophils in an organ or tissue (including blood, esophagus, stomach, duodenum, and ileum) of a patient, with eosinophils greater than or equal to 15 in at least one high power field in a biopsy.

In some embodiments of the present invention, the "patient" includes a subpopulation that is likely to exhibit an elevated level of an EoE-associated biomarker. The EoE-associated biomarker includes, but is not limited to, esophageal eosinophil, eotaxin-3 (CCL26), serum eosinophil peroxidase, chemokine ligand 17 (CCL17), IgE, periostin, IL-5, IL-13, thymic stromal lymphopoietin (TSLP), and eosinophil-derived neurotoxin (EDN).

As used herein, the "allergen" includes any substances, compounds, particles, or compositions that can trigger an allergic reaction in a susceptible individual, including foods such as dairy products, eggs, wheat, legumes, fish, tree nuts, and shellfish.

In certain embodiments of the present invention, the patient described herein is selected from an infant aged 0-1, a child aged 1-5, a juvenile aged 6-12, an adolescent aged 12-18, and an adult aged above 18.

In certain embodiments of the present invention, the IL-4/IL-13 pathway inhibitor is administered in combination with a second therapeutic substance or therapy.

The IL-4/IL-13 pathway inhibitor described herein includes, but is not limited to, an anti-IL-4 antibody, an anti-IL-13 antibody, a bispecific anti-IL-4/IL-13 antibody, an IL-4 receptor (IL-4R) inhibitor, a JAK inhibitor, and a STAT6 inhibitor. In some embodiments, the IL-4/IL-13 pathway inhibitor is an IL-4R inhibitor, such as an anti-IL-4R antibody, more preferably an anti-IL-4RA antibody.

In certain embodiments of the present invention, the method comprises administering to the subject or patient simultaneously or sequentially the second therapeutic agent, wherein preferably, the second therapeutic agent is selected from an IL-1β inhibitor, an IL-5 inhibitor, an IL-8 inhibitor, an IL-9 inhibitor, an IL-13 inhibitor, an IL-17 inhibitor, an IL-25 inhibitor, a JAK inhibitor, a STAT6 inhibitor, a TNF inhibitor, an eotaxin-3 (CCL26) inhibitor, an IgE inhibitor, a prostaglandin D2 inhibitor, an immunosuppressive agent, a corticosteroid, a glucocorticoid, a long-acting β2-agonist, a proton pump inhibitor, a decongestant, an antihistamine and a non-steroidal anti-inflammatory agent (NSAID), an NSAID (non-steroidal anti-inflammatory drug), an antibiotic, an antibacterial agent, an antiviral agent, an antifungal agent, a drug for treating tumors (preferably a chemotherapeutic agent or a growth inhibitor, a targeted therapeutic agent, an antibody-drug conjugate, a T cell expressing chimeric antigen receptors, an antibody or an antigen-binding fragment thereof, an angiogenesis inhibitor, an anti-tumor agent, a cancer vaccine, an adjuvant and a combination thereof, an alkylating agent, an antimetabolite, an antibiotic, a botanical drug, and/or a hormonal drug), or a combination thereof.

In certain embodiments of the present invention, the co-administered therapy includes, but is not limited to, diet therapy and bougienage of oesophagus.

In certain embodiments of the present invention, the IL-4R inhibitor is a dupilumab antibody or a biological equivalent thereof.

In certain embodiments of the present invention, the IL-4R inhibitor is AMG317 or MEDI9314.

In certain embodiments of the present invention, the long-acting β2-agonist is salmeterol or formoterol.

In the present invention, unless otherwise defined, the scientific and technical terms used herein have the meanings generally understood by those skilled in the art. In addition, the laboratory operations of cell culture, molecular genetics, nucleic acid chemistry, and immunology used in the present invention are the routine operations widely used in the corresponding fields. Meanwhile, in order to better understand the present invention, the definitions and explanations of the relevant terms are provided below.

As used herein, the term "antigen-binding region" means a protein or a portion of a protein that specifically binds to a given antigen. For example, a portion of an antibody comprising amino acid residues that interact with an antigen and confer the antibody the specificity and affinity for the antigen is referred to as an "antigen-binding region". The antigen-binding region generally comprises one or more "complementarity determining regions" (CDRs). Certain antigen-binding regions further comprise one or more "framework" regions (FRs). CDRs are amino acid sequences that contribute to antigen-binding specificity and affinity.

As used herein, the term "antibody" refers to an intact immunoglobulin of any isotype or an antigen-binding fragment thereof that can compete with an intact antibody for specifically binding to a target antigen, and includes, for example, chimeric, humanized, fully humanized, and bispecific antibodies or antigen-binding fragments thereof. Such "antibodies" are antigen-binding proteins. An intact antibody generally comprises at least two full-length heavy chains and two full-length light chains, but, in some cases, may comprise fewer chains, such as an antibody naturally existing in camelids that may comprise only a heavy chain. An antibody or an antigen-binding fragment thereof may be derived from a single source only, or may be "chimeric", i.e., different portions of the antibody may be derived from two different sources as further described below. An antibody or an antigen-binding fragment thereof may be produced in hybridomas by recombinant DNA technology, or by enzymatic or chemical cleavage of intact antibodies. Unless otherwise indicated, the term "antibody", in addition to antibodies comprising two full-length heavy chains and two full-length light chains, also includes derivatives, variants, and fragments thereof.

As used herein, the term "antigen-binding fragment" (or abbreviated as "fragment") of an "antibody" or an "immunoglobulin chain" (heavy or light chain) comprises part of an antibody (whether obtained or synthesized) that lacks at least some of the amino acids present in the full length of the antibody but is capable of specifically binding to the antigen. Such fragments are biologically active as they specifically bind to a target antigen and can compete with other antibodies or antigen-binding fragments thereof for specifically binding to a given epitope. In one aspect, such fragments will retain at least one CDR present in the full-length light or heavy chain of the antibody, and in some embodiments, will comprise a single heavy and/or light chain or a portion thereof. Such biologically active fragments can be produced by recombinant DNA technology, or, for example, by enzymatic or chemical cleavage of intact antibodies. Immunologically functional immunoglobulin fragments include, but are not limited to, Fab, Fab', F(ab')₂, Fv, a domain antibody, and a single chain antibody, and can be derived from any mammalian source, including, but not limited to, human, mouse, rat, Camelidae species, and rabbit. It is further contemplated that a functional portion of an antibody disclosed herein, such as one or more CDRs, can be covalently bound to a second protein or a small molecule to generate a therapeutic agent directed to a particular target in the body, thereby having bifunctional therapeutic properties or having an extended serum half-life, such as a fusion protein.

As used herein, the terms "antibody full-length chain", "full-length antibody", "intact antibody", and "whole antibody" are used interchangeably herein to refer to an antibody having a substantially similar structure to a natural antibody structure or having heavy chains in the Fc region as defined herein.

The term "light chain" includes full-length light chains and fragments thereof with sufficient variable region sequences to confer the binding specificity. The full-length light chain comprises a variable region domain VL and a constant region domain CL. The variable region domain of the light chain is at the amino terminus of the polypeptide. Light chains include kappa (κ) and lambda (λ1) chains.

The term "heavy chain" includes full-length heavy chains and fragments thereof with sufficient variable region sequences to confer the binding specificity. The full-length heavy chain comprises a variable region domain VH and 3 constant region domains CH1, CH2, and CH3. The VH domain is at the amino terminus of the polypeptide and the CH domains are at the carboxyl terminus, in which CH3 is closest to the carboxyl terminus of the polypeptide. The heavy chain may be of any isotype, including IgG (including IgG1, IgG2, IgG3, and IgG4 subtypes), IgA (including IgA1 and IgA2 subtypes), IgM, and IgE.

As used herein, the term "Fab" fragment consists of one light chain, CH1 and the variable region of one heavy chain. The heavy chain of an Fab molecule cannot form disulfide bonds with another heavy chain molecule.

As used herein, the term "Fc" region comprises two heavy chain fragments comprising the CH1 and CH2 domains of an antibody. The two heavy chain fragments are held together by two or more disulfide bonds and the hydrophobic interaction of the CH3 domains.

As used herein, the term "Fab' fragment" comprises portions of one light chain and one heavy chain (including the VH domain and the CH1 domain and the region between the CH1 and CH2 domains), such that interchain disulfide bonds can be formed between the two heavy chains of two Fab' fragments to give an F(ab')₂ molecule.

As used herein, the term "F(ab')₂ fragment" comprises two light chains and two heavy chains containing portions of the constant region between the CH1 and CH2 domains such that interchain disulfide bonds are formed between the two heavy chains. Thus, the F(ab')₂ fragment consists of two Fab' fragments held together by disulfide bonds between the two heavy chains.

As used herein, the term "Fv region" comprises the variable regions from the heavy and light chains, but lacks the constant regions.

As used herein, the term "Fd" fragment refers to an antibody fragment consisting of VH and CH1 domains (Ward et al., Nature, 341:544-546 (1989)).

As used herein, the term "dAb" fragment consists of a VH domain (Ward et al., Nature 341:544-546 (1989)).

As used herein, the term "Fab'-SH" is the designation herein for Fab', wherein one or more cysteine residues of the constant domain carry a free thiol group.

As used herein, the term "Fab/c" fragment is an intermediate formed by pepsin digestion of an immunoglobulin, and combines the advantages of Fab and Fc regions, i.e., strong diffusibility and low metabolic clearance *in vivo,* while retaining high affinity (Liu Jianjun, Chinese Journal of Cellular and Molecular Immunology, 1989(4):29-29).

As used herein, the term "single chain antibody" is an Fv molecule in which the heavy and light chain variable regions are connected by a flexible linker to form a single polypeptide chain (which forms an antigen-binding region) (see, e.g., Bird et al., Science, 242:423-426 (1988), and Huston et al., Proc. Natl. Acad. Sci. USA., 90:5879-5883 (1988)). Single chain antibodies are described in detail in International Patent Publication No. WO88/01649 and U.S. Pat. Nos. 4,946,778 and 5,260,203, the disclosures of which are incorporated herein by reference.

As used herein, the term "domain antibody" is an immunofunctional immunoglobulin fragment that comprises only the variable region of the heavy chain or the light chain. In some cases, two or more VH regions are covalently linked by a peptide linker to generate a multivalent domain antibody (particularly a bivalent domain antibody). The two VH regions of the bivalent domain antibody may target the same or different antigens.

As used herein, the term "bivalent antigen-binding protein" or "bivalent antibody" comprises two antigen-binding sites. In some cases, the two binding sites have the same antigen specificity. The bivalent antibody may be bispecific. As used herein, the term "multispecific antigen-binding protein" or "multispecific antibody" is an antigen-binding protein or antibody that targets more than one antigen or epitope.

As used herein, the term "bispecific", "dual-specificity", or "bifunctional" antigen-binding protein or antibody is a hybrid antigen-binding protein or an antibody having two different antigen-binding sites. A bispecific antibody is a multispecific antigen-binding protein or a multispecific antibody, and can be produced by a variety of methods, including, but not limited to, fusion of hybridomas or linkage of Fab' fragments. See, e.g., Songsivilai and Lachmann, 1990, Clin. Exp. Immunol., 79:315-321; Kostelny et al., 1992, J. Immunol., 148:1547-1553. The two binding sites of a bispecific antigen-binding protein or antibody will bind to two different epitopes present in the same or different protein targets.

As used herein, the terms "mAb" and "monoclonal antibody" refer to an antibody or a fragment of an antibody that is derived from a group of highly homologous antibodies, i.e., from a group of identical antibody molecules, except for natural mutations that may occur spontaneously. The monoclonal antibody is highly specific for a single epitope on an antigen. The polyclonal antibody, relative to the monoclonal antibody, generally comprises at least 2 or more different antibodies which generally recognize different epitopes on an antigen. Monoclonal antibodies can generally be obtained using the hybridoma technique first reported by Kohler et al. (Nature, 256:495, 1975), but can also be obtained using recombinant DNA technology (see, e.g., U.S. Patent No. 4,816,567).

As used herein, the term "humanized antibody" refers to an antibody or an antibody fragment obtained when all or part of the CDR regions of a human immunoglobulin (receptor antibody) are replaced by the CDR regions of a non-human antibody (donor antibody), wherein the donor antibody may be a non-human (e.g., mouse, rat, or rabbit) antibody having expected specificity, affinity, or reactivity. In addition, some amino acid residues in the framework regions (FRs) of the receptor antibody can also be replaced by the amino acid residues of corresponding non-human antibodies or by the amino acid residues of other antibodies to further improve or optimize the performance of the antibody. For more details on humanized antibodies, see, e.g., Jones et al., Nature, 321:522-525 (1986); Reichmann et al., Nature, 332:323-329 (1988); Presta, Curr. Op. Struct. Biol., 2:593-596 (1992); and Clark, Immunol. Today, 21: 397-402 (2000). As used herein, the term "epitope" refers to a site on an antigen to which an immunoglobulin or an antibody specifically binds. The "epitope" is also referred to in the art as an "antigenic determinant". The epitope or antigenic determinant generally consists of chemically active surface groups of molecules such as amino acids, carbohydrates, or sugar side chains, and usually has specific three-dimensional structural characteristics and specific charge characteristics. For example, an epitope typically comprises at least 3, 4, 5, 6, 7, 8, 9, 10, 11, 12, 13, 14, or 15 contiguous or non-contiguous amino acids in a unique spatial conformation, and it may be "linear" or "conformational". See, e.g., Epitope Mapping Protocols in Methods in Molecular Biology, Vol. 66, G. E. Morris, Ed. (1996). In a linear epitope, all interaction sites between a protein and an interaction molecule (e.g., an antibody) are located linearly along the primary amino acid sequence of the protein. In a conformational epitope, the interaction sites are located across amino acid residues of a protein that are separated from each other.

The terms "polypeptide" and "protein" are used interchangeably herein to refer to a polymer of amino acid residues. The terms are also used to refer to an amino acid polymer in which one or more amino acid residues are analogs or mimetics of naturally existing amino acids, and to naturally existing amino acid polymers.

The terms may also include, for example, amino acid polymers that have been modified by addition of saccharide residues to form glycoproteins, or have been phosphorylated. Polypeptides and proteins can be produced by naturally existing cells and non-recombinant cells, or they may be produced by genetically engineered or recombinant cells, and comprise a molecule having the amino acid sequence of a native protein or a molecule having deletions, insertions and/or substitutions in one or more amino acids of the native sequence.

In particular, the terms "polypeptide" and "protein" include antibodies, such as anti-human IL-4RA antibodies (also referred to as IL-4RA antibodies), IL-4RA-binding proteins, antibodies or sequences having deletions, insertions, and/or substitutions in one or more amino acids of an antigen-binding protein.

The term "polypeptide fragment" refers to a polypeptide having an amino-terminal deletion, a carboxyl-terminal deletion, and/or an internal deletion as compared to a full-length protein. Such fragments may also comprise modified amino acids as compared to the full-length protein. In certain embodiments, such fragments are about 5 to 500 amino acids in length. For example, the fragments can be at least 5, 6, 8, 10, 14, 20, 50, 70, 100, 110, 150, 200, 250, 300, 350, 400, or 450 amino acids in length. Useful polypeptide fragments include immunologically functional fragments of antibodies, including binding domains. In the case of human IL-4RA antibodies, useful fragments include, but are not limited to, CDR regions, variable domains of heavy or light chains, portions of antibody chains, variable domains just comprising 2 CDRs, or the like.

The terms "human IL-4RA", "hIL-4RA", "human IL-4 receptor A", and "human IL-4 receptor α subunit" are used interchangeably to refer to human interleukin-4 receptor α subunit. Human IL-4RA refers to its mature peptide (Genbank ID: NP_001244336.1). IL-4 and IL-13 are the main endogenous agonists of IL-4RA. Unless otherwise indicated or clear from the context in which the term is used, "IL-4RA" refers to human IL-4RA.

A "derivative" of a polypeptide is a polypeptide (e.g., an antigen-binding protein or an antibody) that is chemically modified in other manners than insertion, deletion or substitution, e.g., by conjugation with another chemical moiety, e.g., a PEG-conjugated polypeptide.

As used herein, the term "specifically bind" refers to a non-random binding reaction between two molecules, such as a reaction between an antibody and an antigen it targets. In some embodiments, an antibody that specifically binds to an antigen (or an antibody that is specific for an antigen) means that the antibody binds to the antigen with an affinity (K_{D}) less than about 10⁻⁵ M, e.g., less than about 10⁻⁶ M, 10⁻⁷ M, 10⁻⁸ M, 10⁻⁹ M, or 10⁻¹⁰ M or less.

As used herein, the term "K_{D}" refers to a dissociation equilibrium constant for a specific antibody-antigen interaction, which is used to describe the binding affinity between the antibody and the antigen. Among the several parameters measured by molecular binding kinetics, the K_{D} value is the dissociation equilibrium constant. In an antibody drug research, it is the parameter characterizing the intensity of the affinity effect of an antibody of interest and the target antigen molecule, and is calculated by the formula: K_{D} = k_{dis}/kₒₙ. A smaller equilibrium dissociation constant indicates a more intensive antibody-antigen binding and a higher affinity between the antibody and the antigen. kₒₙ (association rate constant) is the rate of antigen-antibody complex formation, and a smaller kₒₙ suggests a faster binding of an antibody to an antigen. k_{dis} (dissociation rate constant) is the rate at which an antibody dissociates from an antigen-antibody complex, and a smaller k_{dis} suggests a slower rate for the antibody dissociating from the antigen and a firmer binding between the antibody and the antigen. Generally, an antibody binds to an antigen (e.g., L1 protein) with a dissociation equilibrium constant (K_{D}) of less than about 10⁻⁵ M, e.g., less than about 10⁻⁶ M, 10⁻⁷ M, 10⁻⁸ M, 10⁻⁹ M, or 10⁻¹⁰ M or less, for example, as measured by a BIACORE surface plasmon resonance (SPR) instrument or a Fortebio molecular interaction instrument.

In the present invention, amino acids are generally represented by single-letter and three-letter abbreviations known in the art. For example, alanine can be represented by A or Ala.

As used herein, the terms "hybridoma" and "hybridoma cell line" can be used interchangeably, and when referring to the terms "hybridoma" and "hybridoma cell line", subclones and progeny cells of the hybridoma are also included.

As used herein, the terms "percent sequence identity" and "percent sequence homology" are used interchangeably.

As used herein, the terms "similarity", "sequence similarity", and "identity" refer to the correlation between the sequences of two or more protein or polypeptide molecules, as determined by aligning and comparing the sequences. "Percent identity" refers to the percentage of identical amino acid residues in the molecules compared, and can be calculated based on the size of the smallest molecule to be compared. For such calculations, gaps in the alignment (if any) must be addressed by a particular mathematical model or computer program (i.e., an "algorithm"). The term "substantial identity", when used for polypeptides, means that two peptide sequences, when optimally aligned, for example using the programs GAP or BESTFIT, using default gap weights provided by the programs, have at least 70%, 75%, or 80% sequence identity, at least 90% or 95% sequence identity, or at least 97%, 98%, or 99% sequence identity. In some cases, residue positions that are not identical differ in conservative amino acid substitutions. "Conservative amino acid substitution" is one in which the amino acid residue is substituted with another amino acid residue having a side chain R group that possesses similar chemical properties (e.g., charge or hydrophilicity). Generally, conservative amino acid substitutions will substantially retain the functions and properties of the protein. In cases where two or more amino acid sequences differ from each other by conservative substitutions, the percent sequence identity may be elevated to correct for the conservative nature of the substitution. Methods for making this adjustment are well known to those skilled in the art. See, e.g., Pearson, Methods Mol. Biol., 243:307-31 (1994). Examples of groups of amino acids having side chains with similar chemical properties include: 1) aliphatic hydroxyl side chain: glycine, alanine, valine, leucine, and isoleucine; 2) aliphatic hydroxyl side chain: serine and threonine; 3) amide-containing side chain: asparagine and glutamine; 4) aromatic side chain: phenylalanine, tyrosine, and tryptophan; 5) basic side chain: lysine, arginine, and histidine; 6) acidic side chain: aspartic acid and glutamic acid; and 7) sulfur-containing side chain: cysteine and methionine. The conservative amino acid substitution groups are valine-leucine-isoleucine, phenylalanine-tyrosine, lysine-arginine, alanine-valine, glutamic acid-aspartic acid, and asparagine-glutamine.

Optionally, a conservative substitution is any change with a positive value in the PAM250 log-likelihood matrix disclosed in Gonnet et al., Science, 256:1443-45 (1992), which is incorporated herein by reference. A "moderately conservative" substitution is any change with a non-negative value in the PAM250 log-likelihood matrix.

The sequence identity of polypeptides is usually measured by a sequence analysis software. Protein analysis software aligns sequences using a measure of similarity assigned to different substitutions, deletions, and other modifications (including conservative amino acid substitutions). For example, GCG, including programs such as "Gap" and "Bestfit", (using default parameters specified by the program) can be used to determine sequence homology or sequence identity between closely related polypeptides (e.g., homologous polypeptides from different biological species) or between a wild-type protein and its mutant protein. See, e.g., GCG Version 6.1 (University of Wisconsin, WI). Polypeptide sequences can also be compared using FASTA with default or recommended parameters. See GCG Version 6.10 FASTA (e.g., FASTA2 and FASTA3), which provides alignments for regions of optimal overlap between the challenge and query sequences and percent sequence identities (Pearson, Methods Enzymol., 183:63-98 (1990); Pearson, Methods Mol. Biol., 132:185-219 (2000)). Another preferred algorithm when comparing sequences with a database containing massive sequences from different organisms is the computer program BLAST, in particular, blastp or tblastn (using default parameters provided by the program). See, e.g., Altschul et al., Mol. Biol., 215:403-410 (1990); Al tschul et al., Nucleic Acids Res., 25:3389-402 (1997).

As used herein, the term "effective amount" refers to an amount sufficient to obtain or at least partially obtain a desired effect. For example, the effective amount for preventing diseases (e.g., diseases related to IL-4RA binding to IL-4, such as eosinophilic esophagitis) refers to an amount sufficient to prevent, stop, or delay the onset of diseases (e.g., diseases related to IL-4RA binding to IL-4, such as eosinophilic esophagitis); a therapeutically effective amount against a disease refers to an amount sufficient to cure or at least partially stop a disease and complications thereof in patients suffering from the disease. It is undoubtedly within the ability of those skilled in the art to determine such an effective amount. For example, the amount effective for therapeutic purpose will depend on the severity of the disease to be treated, the overall state of the patient's own immune system, the general condition of the patient such as age, body weight, and gender, the route of administration, and other treatments given concurrently, etc.

### Modes of Administration

The content below is not intended to limit the modes of administration of the antibody or the antigen-binding fragment thereof, the antibody conjugate, the fusion protein, the medicament, or the multi(bi)-specific antibody disclosed herein.

In one embodiment, the antibody or the antigen-binding fragment thereof, the antibody conjugate, the fusion protein, the medicament, or the multi(bi)-specific antibody disclosed herein may be formulated into a pharmaceutical composition suitable for single or multiple doses.

The antibody or the antigen-binding fragment thereof, the antibody conjugate, the fusion protein, the medicament, or the multi(bi)-specific antibody disclosed herein are administered by a variety of routes as appropriate, including, but not limited to, oral or parenteral routes (by intravenous, intramuscular, local, subcutaneous, intraperitoneal, spinal, or other parenteral routes of administration, e.g., by injection or infusion). In some embodiments, the antibody or the antigen-binding fragment thereof, the antibody conjugate, the fusion protein, the medicament, or the multi(bi)-specific antibody disclosed herein is administered orally or by injection, e.g., intravenous or intraperitoneal injection.

As used herein, the phrase "parenteral administration" refers to modes of administration conducted by injection except for enteral and local administration, and includes, but is not limited to, intravenous, intramuscular, intraarterial, intrathecal, intralymphatic, intralesional, intracapsular, intraorbital, intracardiac, intradermal, intraperitoneal, transtracheal, subcutaneous, subepidermal, intraarticular, subcapsular, subarachnoid, intraspinal, epidural and intrasternal injection and infusion, and *in vivo* electroporation.

In certain embodiments, the inhibitor for a relevant factor (e.g., an anti-human IL-4R antibody) is administered by a non-parenteral route, and in some embodiments, it is administered orally. Other non-parenteral routes include local, epidermal, or mucosal routes of administration, for example, intranasal, vaginal, rectal, sublingual, or local administration. Administration may also be performed, e.g., once, multiple times, and/or over one or more extended periods of time. Suitable dosage forms of the antibody or the antigen-binding fragment thereof, the antibody conjugate, the fusion protein, the drug, or the multi(bi)-specific antibody disclosed herein include, but are not limited to, tablet, lozenge, pill, capsule (for example, hard capsule, soft capsule, enteric capsule, and microcapsule), elixir, granule, syrup, injection (intramuscular, intravenous, and intraperitoneal), granule, emulsion, suspension, solution, pulvis, and dosage forms of sustained release formulations for oral or non-oral administration. The medicament disclosed herein contains a pharmaceutically acceptable carrier and/or excipient.

Compared with the prior art, the present invention has the following advantages: The anti-human IL-4RA antibody disclosed herein can bind to human IL-4RA with high affinity, and inhibit relevant cellular biological effects mediated by human IL-4 and human IL-13, such as cell proliferation, IL-4 and IL-13 induced CD23 expression level up-regulation, and the like, by blocking the binding of human IL-4 and human IL-13 to human IL-4RA. The antibody has advantages of high activity, exclusion of species differences, and the like, and can be used in preparing medicaments for blocking the binding of human IL-4 and IL-13 to human IL-4RA and in preparing medicaments for treating or preventing, for example, eosinophilic esophagitis, thus having good application prospect and market value.

### BRIEF DESCRIPTION OF THE DRAWINGS

FIG. 1: Binding activity of 13E5 and dupilumab to antigens human IL-4RA-hFc or human IL-4RA-mFc.
FIG. 2: Binding activity of 13E5H1L1, 13E5H2L2, 13E5H3L3, and dupilumab to antigen IL-4RA-mFc.
FIG. 3: Binding activity of 13E5H4L2, 13E5H4L4, and dupilumab to antigen IL-4RA-mFc.
FIG. 4: Activity of 13E5 and dupilumab in competing with human IL4-N-His for binding to human IL-4RA-hFc.
FIG. 5: Activity of 13E5H1L1, 13E5H2L2, 13E5H3L3, and dupilumab in competing with human IL4-N-His for binding to human IL-4RA-hFc.
FIG. 6: Activity of 13E5H4L2, 13E5H4L4 and dupilumab in competing with human IL4-N-His for binding to human IL-4RA-hFc.
FIG. 7: Activity assay of 13E5H4L4 and dupilumab in competing with human IL4-N-His for binding to human IL-4RA-hFc.
FIG. 8: Affinity constant assay of 13E5H4L4 to human IL-4RA. The antibody concentrations for the curve pairs from top to bottom were 25 nM, 6.25 nM, 3.13 nM, 1.56 nM, 0.78 nM, and 0.39 nM, respectively.
FIG. 9: Affinity constant assay of dupilumab to human IL-4RA. The antibody concentrations for the curve pairs from top to bottom were 12.5 nM, 6.25 nM, 3.13 nM, 1.56 nM, 0.78 nM, and 0.39 nM, respectively.
FIG. 10: Affinity constant assay of 13E5H1L1 to human IL-4RA. The antibody concentrations for the curve pairs from top to bottom were 25 nM, 12.5 nM, 6.25 nM, 3.13 nM, 1.56 nM, 0.78 nM, and 0.39 nM, respectively.
FIG. 11: Affinity constant assay of 13E5H2L2 to human IL-4RA. The antibody concentrations for the curve pairs from top to bottom were 25 nM, 12.5 nM, 6.25 nM, 3.13 nM, 1.56 nM, 0.78 nM, and 0.39 nM, respectively.
FIG. 12: Affinity constant assay of 13E5H3L3 to human IL-4RA. The antibody concentrations for the curve pairs from top to bottom were 25 nM, 12.5 nM, 6.25 nM, 3.13 nM, 1.56 nM, and 0.39 nM, respectively.
FIG. 13: Affinity constant assay of 13E5H4L2 to human IL-4RA. The antibody concentrations for the curve pairs from top to bottom were 12.5 nM, 6.25 nM, 3.13 nM, 1.56 nM, 0.78 nM, and 0.39 nM, respectively.
FIG. 14: Affinity constant assay of dupilumab to human IL-4RA. The antibody concentrations for the curve pairs from top to bottom were 6.25 nM, 3.13 nM, 1.56 nM, 0.78 nM, and 0.39 nM, respectively.
FIG. 15: 13E5H1L1, 13E5H2L2, and dupilumab inhibiting IL-4-induced proliferation of TF-1 cells.
FIG. 16: 13E5H1L1, 13E5H2L2, and dupilumab inhibiting IL-13-induced proliferation of TF-1 cells.
FIG. 17: 13E5H4L2, 13E5H4L4, and dupilumab inhibiting IL-4-induced proliferation of TF-1 cells.
FIG. 18: 13E5H4L2, 13E5H4L4, and dupilumab inhibiting IL-13-induced proliferation of TF-1 cells.
FIG. 19: 13E5H4L4 and dupilumab inhibiting IL-4-induced CD23 expression up-regulation in monocytes.
FIG. 20: 13E5H4L4 and dupilumab inhibiting IL-13-induced CD23 expression up-regulation in monocytes.
FIG. 21: 13E5H4L4 inhibiting chemotaxis of GM-CSF-treated eosinophils towards IL-4.
FIG. 22: 13E5H4L4 inhibiting IL-4-induced secretion of CCL26 by HUVEC cells.
FIG. 23: 13E5H4L4 inhibiting IL-4-induced secretion of CCL26 by A549 cells.
FIG. 24: 13E5H4L4 and dupilumab inhibiting chemotaxis of eosinophils towards IL-4 treated HUVEC culture supernatant.

### DETAILED DESCRIPTION

The embodiments of the present invention will be described in detail below with reference to the examples. Those skilled in the art will appreciate that the following examples are only for illustrating the present invention, and should not be construed as limitations to the scope of the present invention. Examples where the specific technologies or conditions are not specified are performed according to the technologies or conditions described in the publications of the art (e.g., see, Molecular Cloning: A Laboratory Manual, authored by J. Sambrook et al., and translated by Huang Peitang et al., third edition, Science Press) or according to the package insert. Reagents or instruments used are commercially available conventional products if the manufacturers thereof are not specified.

In the following examples of the present invention, BALB/C mice were purchased from Guangdong Medical Laboratory Animal Center.

In the following examples 1-8 of the present invention, the used positive control antibody dupilumab VAB 16F3-1 (hereinafter referred to as dupilumab) was produced by Akeso Biopharma Inc., the sequence of which is referenced to the heavy chain variable region of antibody VAB 16F3-1 (the sequence is set forth in SEQ ID NO: 70; the constant region is an Ig gamma-1 chain C region; ACCESSION No. P01857) and the light chain variable region of antibody VAB 16F3-1 (the coding sequence is set forth in SEQ ID NO: 71; the constant region is an Ig kappa chain C region; ACCESSION No. P01834) described in Patent Application No. PCT/US2007/021210 granted to Regeneron Pharmaceuticals, Inc.

The isotype control antibody hIgG (also written as hIgG4) of the present invention has a heavy chain sequence of SEQ ID NO: 68 and a light chain sequence of SEQ ID NO: 69.

### Example 1 Preparation of Anti-Human IL-4RAAntibody 13E5

### 1. Preparation of hybridoma cell line 13E5

The antigen IL-4RA-mFc for producing anti-IL-4RA antibody was a fusion protein of human IL-4RA mature peptide (Genbank ID: NP_001244336.1) and mFc tag (SEQ ID NO: 67) synthesized by Akeso Biopharma Inc., and was used for immunizing BALB/C mice (purchased from Guangdong Medical Laboratory Animal Center). Spleen cells of immunized BALB/C mice (purchased from Guangdong Medical Laboratory Animal Center) and mouse myeloma cells were fused to form hybridoma cells with reference to existing cell fusion techniques (e.g., Stewart, S.J., "Monoclonal Antibody Production", in Basic Methods in Antibody Production and Characterization, Eds. G.C. Howard and D.R. Bethell, Boca Raton: CRC Press, 2000). The Elisa plate was coated with IL 4RA-hFc protein (IL-4RA is described above, and hFc is a human IgG Fc purification tag, specifically Ig gamma-1 chain C region, Genbank ID: P01857, positions 114-330), which served as the antigen, for indirect ELISA. By screening, hybridoma cells secreting antibodies specifically binding to IL-4RA-hFc were obtained. The hybridoma cells obtained by indirect ELISA screening were subjected to competitive ELISA to select hybridoma cell lines capable of secreting monoclonal antibodies that compete with ligand IL4-N-his (IL4 NCBI Gene ID: AAH70123.1) for binding to IL-4RA-hFc. Two hybridoma cell lines stably secreting anti-human IL-4RA antibodies were obtained by limiting dilution. The above hybridoma cell lines were named as hybridoma cell line LT008, and the monoclonal antibodies secreted by the hybridoma cell line were named as 13E5. Hybridoma cell line LT008 (IL-4RA-13E5) was deposited at China Center for Type Culture Collection (CCTCC) on Jun. 21, 2018 with an accession number CCTCC NO: C2018131, and a preservation address of Wuhan University, Wuhan, China, postal code: 430072.

### 2. Preparation of anti-human IL-4RA antibody 13E5

The cell line LT008 prepared above was cultured with a chemical defined medium (CD medium; containing 1% penicillin-streptomycin) in a 5% CO₂, 37 °C incubator. After 7 days, the supernatant was collected and purified by highspeed centrifugation and vacuum filtration through a microfiltration membrane and through a HiTrap protein A HP column to obtain antibody 13E5.

### Example 2. Sequence Analysis of Anti-Human IL-4RA Antibody 13E5

mRNA was extracted from the cell line LT008 cultured in Example 1 according to the method described in the manual of RNAprep pure Cell/Bacteria Kit (Tiangen, Cat. No. DP430).

cDNA was synthesized according to the manual of Invitrogen SuperScript^{®} III First-Strand Synthesis System for RT-PCR and amplified by PCR.

The PCR-amplified products were directly subjected to TA cloning according to the manual of the pEASY-T1 Cloning Kit (Transgen CT101).

The TA cloning products were directly sequenced, and the sequencing results are as follows:
The nucleotide sequence of the heavy chain variable region is set forth in SEQ ID NO: 1 with a length of 348 bp. The encoded amino acid sequence is set forth in SEQ ID NO: 2 with a length of 116 amino acids, and the sequences of heavy chain CDR1, CDR2, and CDR3 are set forth in SEQ ID NOs: 5, 6, and 7, respectively.

The nucleotide sequence of the light chain variable region is set forth in SEQ ID NO: 3 with a length of 321 bp. The encoded amino acid sequence is set forth in SEQ ID NO: 4 with a length of 107 amino acids, and the sequences of light chain CDR1, CDR2, and CDR3 are set forth in SEQ ID NOs: 8, 9, and 10, respectively.

Example 3: Design and Preparation of Humanized Anti-Human IL-4RA Antibodies 13E5H1L1, 13E5H2L2, 13E5H3L3, 13E5H4L2, and 13E5H4L4
1. Design of light and heavy chain sequences of humanized anti-IL-4RA antibodies 13E5H1L1, 13E5H2L2, 13E5H3L3, 13E5H4L2, and 13E5H4L4

Based on the three-dimensional crystal structure of human IL-4RA protein (Hage T, Reinemer P, Sebald W., Crystals of a 1:1 Complex Between Human Interleukin-4 and the Extracellular Domain of Its Receptor Alpha Chain, Eur. J. Biochem., 1998; 258(2):831-6.) and the sequence of antibody 13E5 obtained in Example 2, the variable region sequences of antibodies 13E5H1L1, 13E5H2L2, 13E5H3L3, 13E5H4L2, and 13E5H4L4 were obtained by computer modeling and mutation design (antibody constant region sequences from NCBI database: the heavy chain constant region is Ig gamma-4 chain C region, ACCESSION No. P01861.1; the light chain constant region is Ig kappa chain C region, ACCESSION No. P01834).

The designed variable region sequences are as follows:
(1) Heavy and light chain sequences of humanized monoclonal antibody 13E5H1L1
   The nucleotide sequence of the heavy chain variable region is set forth in SEQ ID NO: 11 with a length of 348 bp. The encoded amino acid sequence is set forth in SEQ ID NO: 12 with a length of 116 aa, and the sequences of heavy chain CDR1, CDR2, and CDR3 are set forth in SEQ ID NOs: 5, 6, and 7, respectively. The nucleotide sequence of the light chain variable region is set forth in SEQ ID NO: 13 with a length of 321 bp. The encoded amino acid sequence is set forth in SEQ ID NO: 14 with a length of 107 aa, and the sequences of light chain CDR1, CDR2, and CDR3 are set forth in SEQ ID NOs: 8, 9, and 10, respectively.
(2) Heavy and light chain sequences of humanized monoclonal antibody 13E5H2L2
   The nucleotide sequence of the heavy chain variable region is set forth in SEQ ID NO: 15 with a length of 348 bp. The encoded amino acid sequence is set forth in SEQ ID NO: 16 with a length of 116 aa, and the sequences of heavy chain CDR1, CDR2, and CDR3 are set forth in SEQ ID NOs: 5, 6, and 7, respectively. The nucleotide sequence of the light chain variable region is set forth in SEQ ID NO: 17 with a length of 321 bp. The encoded amino acid sequence is set forth in SEQ ID NO: 18 with a length of 107 aa, and the sequences of light chain CDR1, CDR2, and CDR3 are set forth in SEQ ID NOs: 8, 9, and 10, respectively.
(3) Heavy and light chain sequences of humanized monoclonal antibody 13E5H3L3
   The nucleotide sequence of the heavy chain variable region is set forth in SEQ ID NO: 19 with a length of 348 bp. The encoded amino acid sequence is set forth in SEQ ID NO: 20 with a length of 116 aa, and the sequences of heavy chain CDR1, CDR2, and CDR3 are set forth in SEQ ID NOs: 5, 6, and 7, respectively. The nucleotide sequence of the light chain variable region is set forth in SEQ ID NO: 21 with a length of 321 bp. The encoded amino acid sequence is set forth in SEQ ID NO: 22 with a length of 107 aa, and the sequences of light chain CDR1, CDR2, and CDR3 are set forth in SEQ ID NOs: 8, 9, and 10, respectively.
(4) Heavy and light chain sequences of humanized monoclonal antibody 13E5H4L4
   The nucleotide sequence of the heavy chain variable region is set forth in SEQ ID NO: 23 with a length of 348 bp. The encoded amino acid sequence is set forth in SEQ ID NO: 24 with a length of 116 aa, and the sequences of heavy chain CDR1, CDR2, and CDR3 are set forth in SEQ ID NOs: 5, 6, and 7, respectively. The nucleotide sequence of the light chain variable region is set forth in SEQ ID NO: 25 with a length of 321 bp. The encoded amino acid sequence is set forth in SEQ ID NO: 26 with a length of 107 aa, and the sequences of light chain CDR1, CDR2, and CDR3 are set forth in SEQ ID NOs: 8, 9, and 10, respectively.
(5) Heavy and light chain sequences of humanized monoclonal antibody 13E5H4L2

The nucleotide sequence of the heavy chain variable region is set forth in SEQ ID NO: 23.

The encoded amino acid sequence is set forth in SEQ ID NO: 24.

The nucleotide sequence of the light chain variable region is set forth in SEQ ID NO: 17.

The encoded amino acid sequence is set forth in SEQ ID NO: 18.

### 2. Preparation of humanized antibodies 13E5H1L1, 13E5H2L2, 13E5H3L3, 13E5H4L2, and 13E5H4L4

The heavy chain constant regions were all Ig gamma-4 chain C region, ACCESSION: P01861.1; the light chain constant regions were all Ig kappa chain C region, ACCESSION: P01834.

The heavy chain variable region cDNA and the light chain variable region cDNA of 13E5H1L1, the heavy chain variable region cDNA and the light chain variable region cDNAof 13E5H2L2, the heavy chain variable region cDNA and the light chain variable region cDNA of 13E5H3L3, the heavy chain variable region cDNA and the light chain variable region cDNA of 13E5H4L2, and the heavy chain variable region cDNA and the light chain variable region cDNA of 13E5H4L4 were separately cloned into a pUC57 simple (supplied by Genscript Biotech Corporation) vector to obtain pUC57 simple-13E5H1, pUC57simple-13E5L1, pUC57simple-13E5H2, pUC57simple-13E5L2, pUC57simple-13E5H3, pUC57simple-13E5L3, pUC57simple-13E5H4, and pUC57simple-13E5L4, respectively. The variable region fragments were acquired by enzyme digestion according to the standard techniques described in Molecular Cloning: A Laboratory Manual (Second Edition), and subcloned into a pcDNA3.1 vector containing the corresponding heavy or light chain constant region fragment (the heavy and light chain constant regions were cloned into the vector pcDNA3.1 by restriction enzyme HindIII & EcoRI digestion) to obtain pcDNA3.1-13E5H1, pcDNA3.1-13E5L1, pcDNA3.1-13E5H2, pcDNA3.1-13E5L2, pcDNA3.1-13E5H3, pcDNA3.1-13E5L3, pcDNA3.1-13E5H4, and pcDNA3.1-13E5L4. Then the recombinant plasmids pairs containing the corresponding light and heavy chains (pcDNA3.1-13E5H1 and pcDNA3.1-13E5L1, pcDNA3.1-13E5H2 and pcDNA3.1-13E5L2, pcDNA3.1-13E5H3 and pcDNA3.1-13E5L3, pcDNA3.1-13E5H4 and pcDNA3.1-13E5L4, and pcDNA3.1-13E5H4 and pcDNA3.1-13E5L2) were separately co-transfected into 293F cells, and the culture solutions were collected and purified. After the sequences were verified, endotoxin-free expression plasmids were prepared, and were transiently transfected into HEK293 cells for antibody expression. After 7 days of culture, the cell cultures were collected, and subjected to affinity purification on a Protein A column to obtain humanized antibodies 13E5H1L1, 13E5H2L2, 13E5H3L3, 13E5H4L2, and 13E5H4L4.

Example 4: Assay for Binding Activity of Antibodies to Antigens by ELISA

### 1. Binding activity of murine antibody 13E5 and humanized antibodies 13E5H1L1, 13E5H2L2, 13E5H3L3, 13E5H4L2, and 13E5H4L4 to antigens human IL-4RA-hFc or human IL-4RA-mFc

1.1. The binding activity of antibodies 13E5, 13E5H1L1, 13E5H2L2, 13E5H3L3, 13E5H4L2, and 13E5H4L4 to antigens human IL-4RA-hFc or human IL-4RA-mFc was detected by indirect ELISA.

Procedures: The Elisa plates were coated with human IL-4RA-hFc or human IL-4RA-mFc and were incubated, and the target antibodies were added after blocking. Goat anti-mouse IgG (H+L) and HRP (purchased from Jackson ImmunoResearch Inc., Cat No. 109-035-062), or goat anti-human IgG and HRP (purchased from Jackson ImmunoResearch Inc., Cat No. 109-035-088) were added. The plates were incubated and washed before TMB (Neogen, 308177) was added for chromogenic reaction. At the end of the reaction, the absorbance at 450 nm was measured by a microplate reader. The data were analyzed and processed by SoftMax Pro 6.2.1.

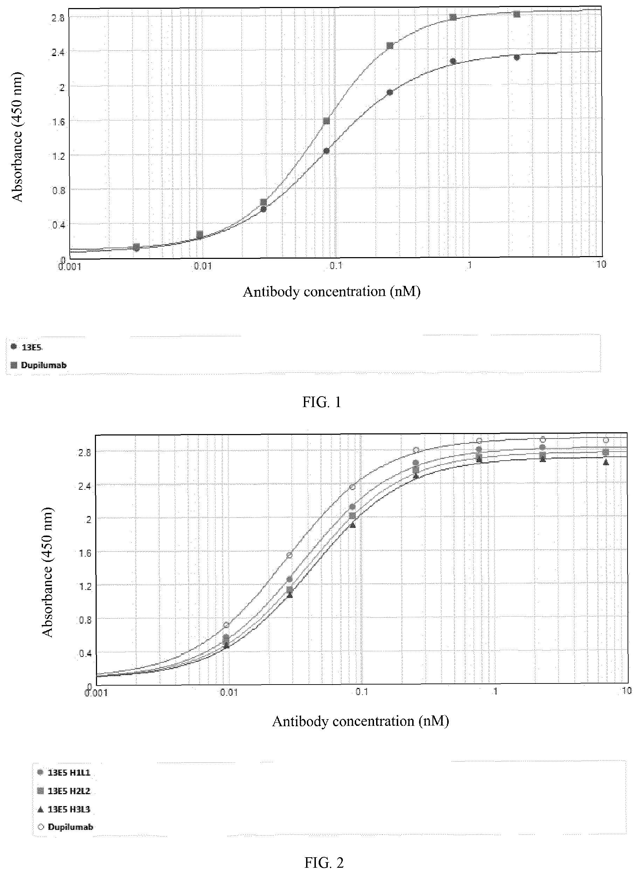

The readings at 450 nm show that antibodies 13E5, 13E5H1L1, 13E5H2L2, 13E5H3L3, 13E5H4L2, and 13E5H4L4 are all capable of effectively binding to antigens human IL-4RA-hFc or human IL-4RA-mFc in a dose-dependent manner. By 4-parameter logistic regression of absorbance vs. antibody concentration, as shown in Tables 1, 2, and 3, 13E5, 13E5H1L1, 13E5H2L2, 13E5H3L3, 13E5H4L2, and 13E5H4L4 are all capable of effectively binding to antigens human IL-4RA-hFc or human IL-4RA-mFc, and demonstrate comparable binding activities to that of the positive control antibody dupilumab against the same antigen (FIGs. 1, 2, and 3).

**Table 1. Binding activity of 13E5 and dupilumab to antigens human IL-4RA-hFc or human IL-4RA-mFc.**

| Antibody dilution (µg/mL) | Antigen coating (1 µg/mL) | | | |
|---|---|---|---|---|
| | IL-4RA-hFc | | IL-4RA-mFc | |
| | 13E5 | | Dupilumab | |
| 0.333 | 2.300 | 2.313 | 2.758 | 2.858 |
| 1:3 | 2.318 | 2.215 | 2.748 | 2.801 |
| 1:9 | 1.923 | 1.878 | 2.388 | 2.500 |
| 1:27 | 1.251 | 1.207 | 1.469 | 1.679 |
| 1:81 | 0.558 | 0.563 | 0.599 | 0.673 |
| 1:243 | 0.244 | 0.237 | 0.250 | 0.281 |
| 1:729 | 0.112 | 0.113 | 0.126 | 0.137 |
| 0 | 0.047 | 0.053 | 0.076 | 0.069 |
| Secondary antibody | Goat anti-mouse IgG (H+L), HRP (1:5000) | | Goat anti-human IgG (H+L), HRP (1:5000) | |
| EC₅₀ (nM) | 0.084 | | 0.077 | |

**Table 2. Binding activity of 13E5H1L1, 13E5H2L2, 13E5H3L3, and dupilumab to antigen IL-4RA-mFc.**

| Antibody dilution (µg/mL) | Antigen coating: IL-4RA-mFc | | | | | | | |
|---|---|---|---|---|---|---|---|---|
| | 13E5H1L1 | | 13E5H2L2 | | 13E5H3L3 | | Dupilumab | |
| 1 | 2.716 | 2.797 | 2.768 | 2.754 | 2.642 | 2.656 | 2.901 | 2.921 |
| 1:3 | 2.834 | 2.825 | 2.686 | 2.770 | 2.679 | 2.688 | 2.928 | 2.923 |
| 1:9 | 2.802 | 2.798 | 2.692 | 2.735 | 2.658 | 2.717 | 2.909 | 2.921 |
| 1:27 | 2.684 | 2.613 | 2.551 | 2.578 | 2.492 | 2.498 | 2.799 | 2.801 |
| 1:81 | 2.116 | 2.122 | 1.972 | 2.043 | 1.918 | 1.891 | 2.358 | 2.358 |
| 1:243 | 1.214 | 1.297 | 1.104 | 1.153 | 1.069 | 1.080 | 1.551 | 1.544 |
| 1:729 | 0.571 | 0.566 | 0.499 | 0.528 | 0.469 | 0.464 | 0.717 | 0.705 |
| 0 | 0.056 | 0.059 | 0.055 | 0.056 | 0.056 | 0.056 | 0.056 | 0.093 |
| Secondary antibody | Goat anti-human IgG (H+L), HRP (1:5000) | | | | | | | |
| EC₅₀ (nM) | 0.035 | | 0.040 | | 0.042 | | 0.028 | |

**Table 3. Binding activity of 13E5H4L2, 13E5H4L4, and dupilumab to antigen IL-4RA-mFc.**

| Antibody dilution (µg/mL) | Antigen coating: IL-4RA-mFc | | | | | |
|---|---|---|---|---|---|---|
| | 13E5H4L2 | | 13E5H4L4 | | Dupilumab | |
| 0.333 | 2.733 | 2.772 | 2.798 | 2.879 | 2.850 | 2.893 |
| 1:3 | 2.730 | 2.775 | 2.766 | 2.730 | 2.676 | 2.673 |
| 1:9 | 2.359 | 2.406 | 2.370 | 2.392 | 2.482 | 2.408 |
| 1:27 | 1.492 | 1.523 | 1.474 | 1.608 | 1.637 | 1.722 |
| 1:81 | 0.673 | 0.707 | 0.709 | 0.778 | 0.861 | 0.816 |
| 1:243 | 0.305 | 0.293 | 0.317 | 0.313 | 0.335 | 0.336 |
| 1:729 | 0.145 | 0.134 | 0.146 | 0.147 | 0.147 | 0.147 |
| 0 | 0.063 | 0.063 | 0.064 | 0.064 | 0.067 | 0.064 |
| Secondary antibody | Goat anti-human IgG (H+L), HRP (1:5000) | | | | | |
| EC₅₀ (nM) | 0.079 | | 0.079 | | 0.066 | |

### 1.2. The activity of antibodies 13E5, 13E5H1L1, 13E5H2L2, 13E5H3L3, 13E5H4L2, and 13E5H4L4 for blocking the binding between IL4-N-his and the antigen IL-4RA-hFc was detected by competitive ELISA

The EC₅₀ (median effect concentration) of 13E5, 13E5H1L1, 13E5H2L2, 13E5H3L3, 13E5H4L2, and 13E5H4L4 in competing with ligand human IL4-N-His for binding to the target antigen human IL-4RA-hFc was assayed by ELISA, so as to investigate the activity of 13E5, 13E5H1L1, 13E5H2L2, 13E5H3L3, 13E5H4L2, and 13E5H4L4 for blocking the binding of the ligand to the target antigen human IL-4RA-hFc.

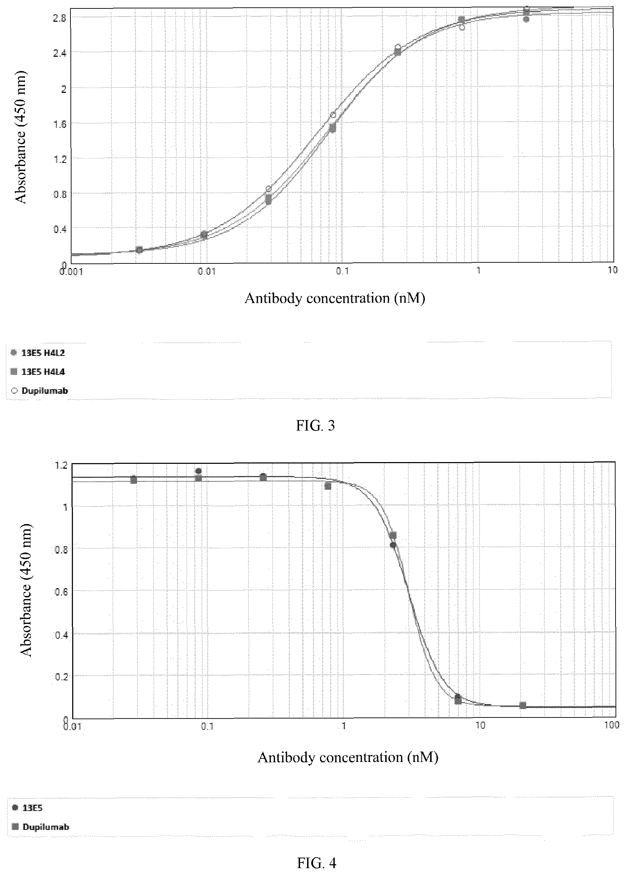

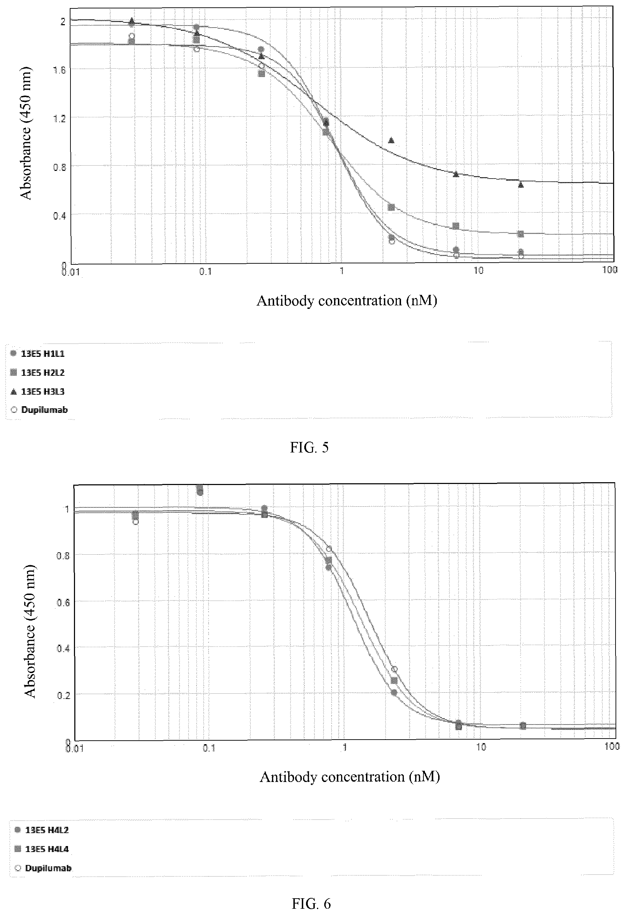

The Elisa plates were coated with human IL-4RA-hFc and blocked before the target antibodies were added. An equal volume of human IL4-N-His (synthesized by Akeso Biopharma Inc.) was added, and the mixture was well mixed and incubated. After the plates were washed, mouse anti-His, HRP (purchased from CoWin Biosciences, Cat No. CW0285A) was added for incubation. The plates were subjected to another wash. A chromogenic reaction was performed by adding TMB (Neogen, 308177) and was then terminated. The Elisa plates were put into a microplate reader immediately, and the OD value of each well in the Elisa plates was read at 450 nm. The data were analyzed and processed by SoftMax Pro 6.2.1. The results are shown in Tables 4, 5, and 6. By 4-parameter logistic regression of absorbance vs. antibody concentration, the blocking EC₅₀ of the antibodies was calculated. As can be seen from FIGs. 4, 5, and 6, 13E5, 13E5H1L1, 13E5H2L2, 13E5H4L2, and 13E5H4L4 are all capable of effectively blocking the binding of ligand human IL4-N-His to the antigen human IL-4RA-hFc in a dose-dependent manner. Antibodies 13E5H1L1, 13E5H2L2, 13E5H4L2, and 13E5H4L4 all demonstrate superior activity in competing with human IL4-N-His for binding to human IL-4RA-hFc as compared to that of the positive control antibody dupilumab for the same antigen.

**Table 4. Activity assay of 13E5 and dupilumab in competing with human IL4-N-His for binding to human IL-4RA-hFc**

| Antibody dilution (µg/mL) | Antigen binding: IL-4RA-hFc | | | |
|---|---|---|---|---|
| | 13E5 | | Dupilumab | |
| 3 | 0.053 | 0.053 | 0.054 | 0.052 |
| 1:3 | 0.093 | 0.100 | 0.074 | 0.076 |
| 1:9 | 0.790 | 0.832 | 0.837 | 0.877 |
| 1:27 | 1.080 | 1.095 | 1.056 | 1.123 |
| 1:81 | 1.108 | 1.164 | 1.100 | 1.149 |
| 1:243 | 1.120 | 1.199 | 1.126 | 1.126 |
| 1:729 | 1.125 | 1.129 | 1.076 | 1.158 |
| 0 | 1.136 | 1.156 | 1.123 | 1.093 |
| IL4-N-his | | | | |
| Secondary antibody | Mouse anti-his, HRP (1:4000) | | | |
| EC₅₀ (nM) | 3.000 | | 3.033 | |

**Table 5. Activity assay of 13E5H1L1, 13E5H2L2, 13E5H3L3, and dupilumab in competing with human IL4-N-His for binding to human IL-4RA-hFc**

| Antibody dilution (µg/mL) | Antigen coating: IL-4RA-hFc | | | | | | | |
|---|---|---|---|---|---|---|---|---|
| | 13E5H1L1 | | 13E5H2L2 | | 13E5H3L3 | | Dupilumab | |
| 3 | 0.070 | 0.078 | 0.230 | 0.216 | 0.645 | 0.620 | 0.050 | 0.049 |
| 1:3 | 0.095 | 0.103 | 0.282 | 0.306 | 0.727 | 0.708 | 0.059 | 0.057 |
| 1:9 | 0.179 | 0.225 | 0.443 | 0.455 | 1.047 | 0.955 | 0.186 | 0.159 |
| 1:27 | 0.921 | 1.415 | 1.048 | 1.096 | 1.450 | 0.845 | 1.230 | 1.053 |
| 1:81 | 1.468 | 2.037 | 1.562 | 1.543 | 1.705 | 1.686 | 1.680 | 1.546 |
| 1:243 | 1.705 | 2.157 | 1.776 | 1.888 | 1.932 | 1.846 | 1.774 | 1.743 |
| 1:729 | 1.746 | 2.182 | 1.792 | 1.845 | 2.060 | 1.933 | 1.889 | 1.841 |
| 0 | 1.793 | 2.243 | 1.741 | 1.776 | 2.056 | 1.912 | 1.828 | 1.806 |
| IL4-N-his | | | | | | | | |

| Secondary antibody | | | | Mouse anti-his, HRP (1:4000) | | | | |
|---|---|---|---|---|---|---|---|---|
| EC₅₀ (nM) | 0.875 | | 0.819 | | 0.636 | | 0.943 | |

**Table 6. Activity assay of 13E5H4L2, 13E5H4L4, and dupilumab in competing with human IL4-N-His for binding to human IL-4RA-hFc**

| Antibody dilution (µg/mL) | Antigen coating: IL-4RA-hFc | | | | | |
|---|---|---|---|---|---|---|
| | 13E5H4L2 | | 13E5H4L4 | | Dupilumab | |
| 3 | 0.056 | 0.059 | 0.054 | 0.051 | 0.054 | 0.057 |
| 1:3 | 0.068 | 0.065 | 0.053 | 0.052 | 0.052 | 0.053 |
| 1:9 | 0.202 | 0.197 | 0.228 | 0.274 | 0.292 | 0.308 |
| 1:27 | 0.697 | 0.780 | 0.752 | 0.786 | 0.771 | 0.871 |
| 1:81 | 0.996 | 0.991 | 0.903 | 1.030 | 0.971 | 0.966 |
| 1:243 | 1.082 | 1.047 | 1.030 | 1.135 | 1.050 | 1.073 |
| 1:729 | 0.964 | 0.988 | 0.950 | 0.978 | 0.918 | 0.964 |
| 0 | 0.935 | 0.960 | 0.932 | 0.898 | 0.896 | 0.956 |
| IL4-N-his | | | | | | |

| Secondary antibody | Mouse anti-his, HRP (1:4000) | | | | | |
|---|---|---|---|---|---|---|
| EC₅₀ (nM) | 1.150 | | 1.328 | | 1.546 | |

### 2. Binding activity of humanized antibody 13E5H4L4 to antigen IL-4RA-hFc

2.1. The binding activity of antibody 13E5H4L4 for blocking the binding between IL4-N-his and the antigen IL-4RA-hFc was detected by competitive ELISA.

The EC₅₀ (median effect concentration) of 13E5H4L4 in competing with ligand human IL4-N-His for binding to the target antigen human IL-4RA-hFc was assayed by ELISA, so as to investigate the activity of 13E5H4L4 for blocking the binding of the ligand to the target antigen human IL-4RA-hFc.

The Elisa plates were coated with human IL-4RA-hFc and blocked before the target antibodies were added. An equal volume of human IL4-N-His (synthesized by Akeso Biopharma Inc.) was added, and the mixture was well mixed and incubated. After the plates were washed, mouse anti-his, HRP (purchased from CoWin Biosciences, Cat No. CW0285A) was added for incubation. The plates were subjected to another wash. A chromogenic reaction was performed by adding TMB (Neogen, 308177) and was then terminated. The Elisa plates were put into a microplate reader immediately, and the OD value of each well in the Elisa plates was read at 450 nm. The data were analyzed and processed by SoftMax Pro 6.2.1. The results are shown in Table 7.

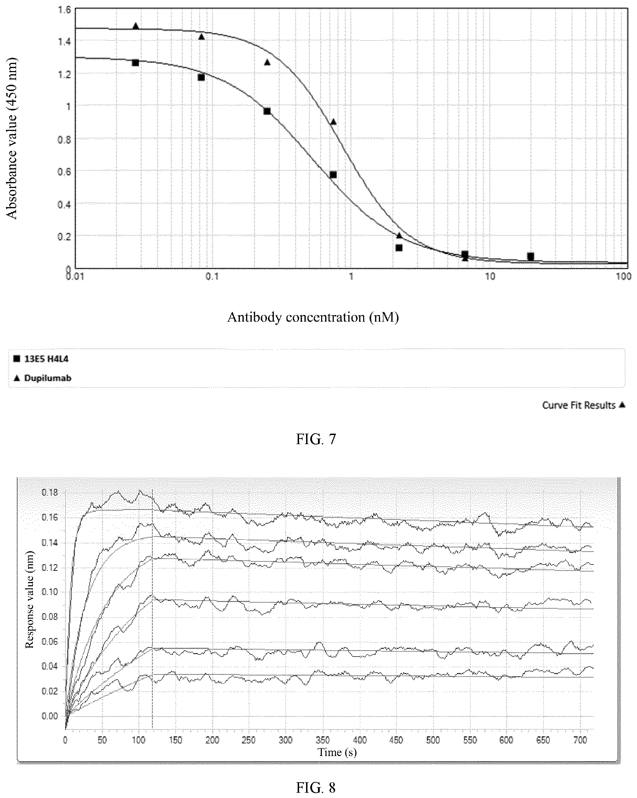

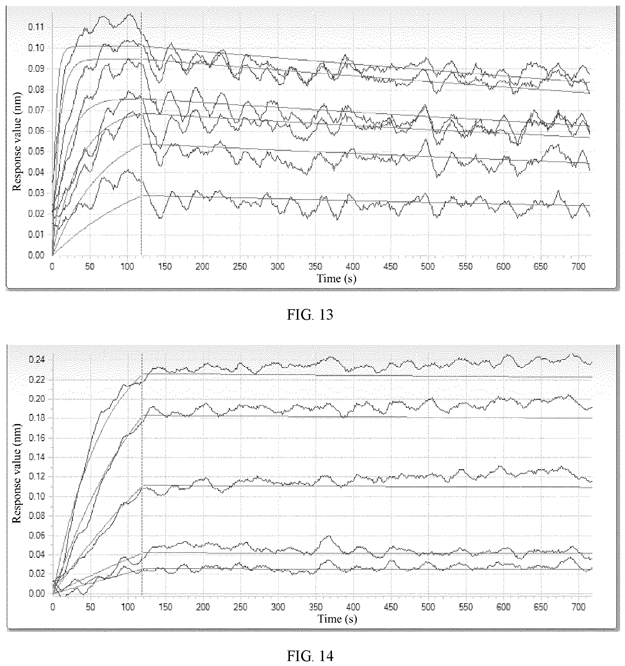

By 4-parameter logistic regression of absorbance vs. antibody concentration, the blocking EC₅₀ of the antibodies was calculated. As can be seen from FIG. 7, 13E5H4L4 is capable of effectively blocking the binding of the ligand human IL4-N-His to the antigen human IL-4RA-hFc in a dose-dependent manner. Antibody 13E5H4L4 demonstrates superior activity in competing with human IL4-N-His for binding to human IL-4RA-hFc as compared to that of the positive control antibody dupilumab for the same antigen.

**Table 7. Activity assay of 13E5H4L4 and dupilumab in competing with human IL4-N-His for binding to human IL-4RA-hFc**

| Antibody dilution (µg/mL) | Antigen coating: IL-4RA-hFc (2 µg/mL) | | | |
|---|---|---|---|---|
| | 13E5H4L4 | | Dupilumab | |
| 3 | 0.065 | 0.074 | 0.059 | 0.059 |
| 1:3 | 0.097 | 0.071 | 0.059 | 0.060 |
| 1:9 | 0.123 | 0.119 | 0.201 | 0.197 |
| 1:27 | 0.554 | 0.594 | 0.853 | 0.948 |
| 1:81 | 0.944 | 0.976 | 1.241 | 1.287 |
| 1:243 | 1.137 | 1.200 | 1.443 | 1.400 |
| 1:729 | 1.252 | 1.269 | 1.534 | 1.441 |
| 0 | 1.328 | 1.349 | 1.559 | 1.482 |
| IL4-N-his:0.15µg/ml | | | | |

| Secondary antibody | Mouse anti-his, HRP (1:4000) | | | |
|---|---|---|---|---|
| EC₅₀ (nM) | 0.532 | | 0.865 | |

### Example 5 Determination of Affinity Constants of Humanized Antibodies 13E5H1L1, 13E5H2L2, 13E5H3L3, 13E5H4L2, and 13E5H4L4 for Human IL-4RA

By Fortebio molecular interaction instrument, the affinity constants of humanized antibodies 13E5H1L1, 13E5H2L2, 13E5H3L3, 13E5H4L2, 13E5H4L4, and dupilumab for human IL-4RA were determined.

5 µg/mL antigen was immobilized on an AMC sensor by 60 s of incubation. The sensor was then equilibrated in PBST for 300 s and incubated for 120 s in the antibodies at 0.39-25 nM (two-fold serial dilution) for binding the antibodies to the antigen immobilized on the sensor. The remaining antibodies were dissociated with the antigen by a 600-second incubation in PBST. The sensor was refreshed in 10 mM glycine, pH 1.7. The data were analyzed by 1:1 model fitting to obtain affinity constants.

The affinity constants of humanized antibody 13E5H4L4 and dupilumab (as a positive control) for human IL-4RA are shown in Table 8 and FIGs. 8-9.

**Table 8. Affinity constant assay of 13E5H4L4 and dupilumab for human IL-4RA**

| Name of antibody | K_{D} (M) | kon (1/Ms) | kon Error | kdis (1/s) | kdis Error | Rmax (nm) |
|---|---|---|---|---|---|---|
| 13E5H4L4 | 2.63E-11 | 5.56E+06 | 1.60E+05 | 1.46E-04 | 9.74E-06 | 0.1370-0.1667 |
| Dupilumab | 1.14E-11 | 7.08E+06 | 2.15E+05 | 8.10E-05 | 1.13E-05 | 0.1325-0.2184 |

| | | | | | | |
|---|---|---|---|---|---|---|
| K_{D} is the affinity constant; K_{D} = kdis/kon. | | | | | | |

The results show that the humanized antibody 13E5H4L4 has a high binding capacity to human IL-4RA comparable to that of the positive control antibody dupilumab for the same target.

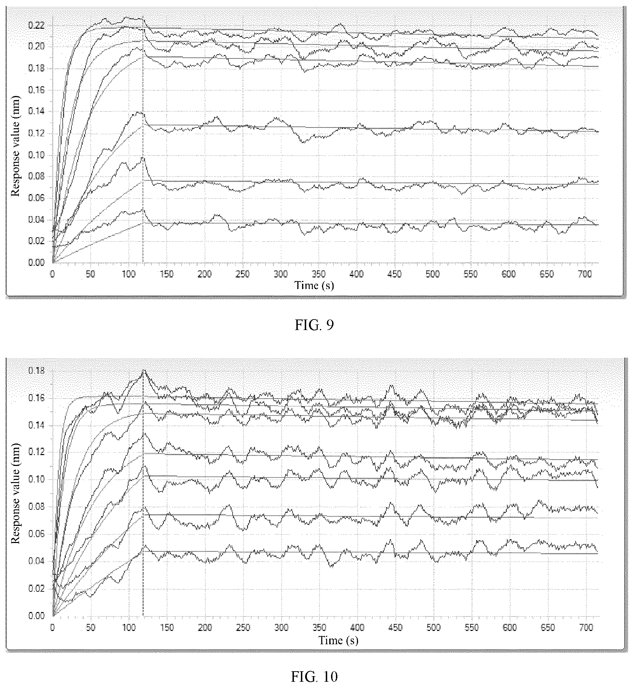

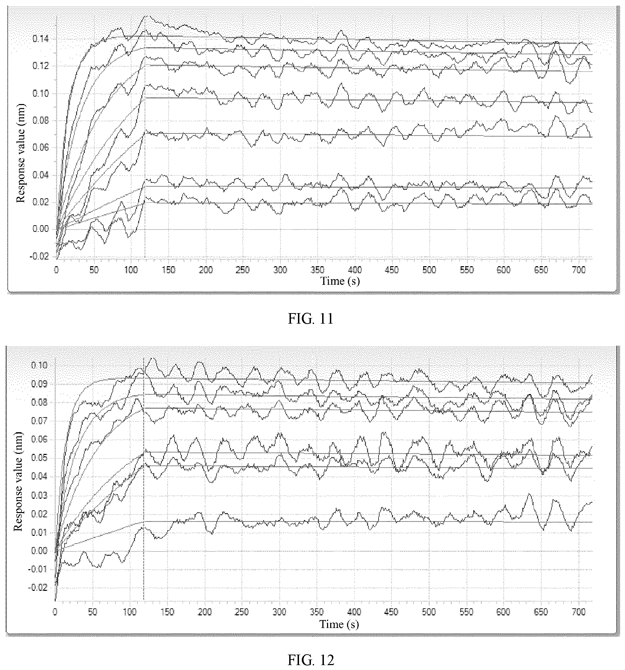

The affinity constants of humanized antibodies 13E5H1L1, 13E5H2L2, 13E5H3L3, 13E5H4L2, and dupilumab (as a positive control) for human IL-4RA are shown in Table 9 and FIGs. 10-14.

**Table 9. Affinity constant assay of 13E5H1L1, 13E5H2L2, 13E5H3L3, 13E5H4L2, and dupilumab for human IL-4RA**

| Antibody | K_{D} (M) | kon (1/Ms) | kon Error | kdis (1/s) | kdis Error | Rmax (nm) |
|---|---|---|---|---|---|---|
| 13E5H1L1 | 1.00E-11 | 5.78E+06 | 1.94E+05 | 5.79E-05 | 1.12E-05 | 0.1347-0.2008 |
| 13E5H2L2 | 2.83E-11 | 2.44E+06 | 9.77E+04 | 6.90E-05 | 1.44E-05 | 0.1377-0.1944 |
| 13E5H3L3 | 1.82E-11 | 2.79E+06 | 1.46E+05 | 5.09E-05 | 1.90E-05 | 0.0824-0.1332 |
| 13E5H4L2 | 1.79E-11 | 1.78E+07 | 1.16E+06 | 3.18E-04 | 2.15E-05 | 0.0521-0.1012 |
| Dupilumab | 1.17E-11 | 2.35E+06 | 1.68E+05 | 2.75E-05 | 1.73E-05 | 0.2131-0.3131 |

| | | | | | | |
|---|---|---|---|---|---|---|
| K_{D} is the affinity constant; K_{D} = kdis/kon. | | | | | | |

The results show that the affinity of the humanized antibody 13E5H1L1 for the antigen is superior to that of the positive control antibody dupilumab for the same target; the dissociation rate constants kdis of 13E5H1L1 and 13E5H2L2 with human IL-4RA are less than that of the positive control antibody dupilumab for the same target, suggesting more stable binding of 13E5H2L2 to human IL-4RA. The binding rate constants of 13E5H1L1 and 13E5H4L2 to the antigen human IL-4RA are greater than that of the positive control antibody dupilumab for the same target, suggesting faster binding of 13E5H1L1 and 13E5H4L2 to the antigen human IL-4RA than dupilumab.

### Example 6. Cellular Bioactivity Assay

Cellular bioactivity of 13E5H1L1, 13E5H2L2, 13E5H4L2, and 13E5H4L4 for blocking the TF-1 cell proliferation induced by human IL-4 and human IL-13 was detected. The procedures are as follows:
TF-1 cells (purchased from American Type Culture Collection, Cat. No. CRL-2003) were cultured (complete medium: RPMI 1640 + 10% FBS + 2.5 g/L glucose + 2 ng/mL GM-CSF). On the day of assay, TF-1 cells were collected after centrifugation, resuspended in a medium free of GM-CSF, and counted. The cells were inoculated into 96-well plates at 20,000 cells/well. The treatment was given as designed: the final concentrations of the antibodies were 0.05, 0.5, and 5 nM; three concentrations 0.041, 0.41, and 4.1 nM were set for IL-4, and the antibody group employed 0.41 nM IL-4; three concentrations 1.58, 15.8, and 79 nM were set for IL-13, and the antibody group employed 15.8 nM IL-13. After administration, the 96-well plates were incubated in a 5% carbon dioxide incubator at 37 °C for 72 h. After the 72 h, CCK8 reagent was added according to the instruction of CCK8 kit (purchased from Dojindo Laboratories, Japan, Cat No. CK04). The mixture was mixed well and incubated at 37 °C for 3-4 h in a 5% carbon dioxide incubator, and the OD value at 450 nm was read. A curve of OD vs. cell number was plotted by seeding serially diluted TF-1 cells into 96-well plates, adding CCK8 reagent, incubating the plates in a 5% carbon dioxide incubator at 37 °C for 3-4 h, and reading the OD value at 450 nm. The cell numbers of the groups were calculated according to the OD value, and GraphPad Prism 5 was used for plotting.

The isotype control antibody was human anti-hen egg lysozyme IgG (anti-HEL, i.e., human IgG, or hIgG) derived from the variable region sequence of the Fab F10.6.6 sequence in Affinity Maturation Increases the Stability and Plasticity of the Fv Domain of Anti-Protein Antibodies (Acierno et al., J Mol Biol., 2007, 374(1):130-46). The isotype was synthesized by Akeso Biopharma Inc.

Nanjing Genscript Biotech Corporation was entrusted with codon optimization and gene synthesis for the heavy and light chain (complete sequence or variable region) genes of the human IgG antibodies. With reference to the standard techniques described in Molecular Cloning: A Laboratory Manual (Second Edition), the heavy and light chain genes were subcloned into the antibody heavy and light chain expression vectors (both were pcDNA3.1 vector) of mammalian expression system by standard molecular cloning techniques such as enzyme digestion, DNA gel recovery, ligation and transformation, colony PCR or enzyme digestion identification. The heavy and light chain genes of the recombinant expression vector were further sequenced and analyzed (the heavy chain sequence is set forth in SEQ ID NO: 68, and the light chain sequence is set forth in SEQ ID NO: 69). After the sequences were verified to be correct, endotoxin-free expression plasmids were prepared in a large scale, and the heavy and light chain expression plasmids were transiently co-transfected into HEK293 cells for recombinant antibody expression. After 7 days of culture, the cell cultures were collected and subjected to affinity purification on a rProtein A column (GE), and the quality of the resulting antibody sample was determined using SDS-PAGE and SEC-HPLC standard analysis techniques.

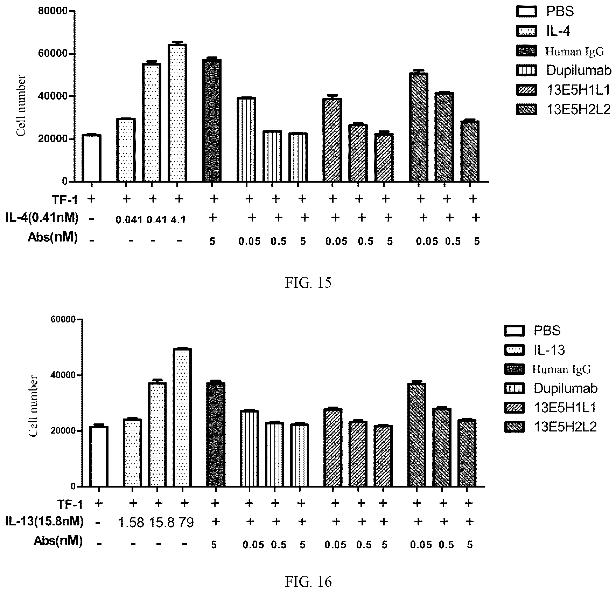

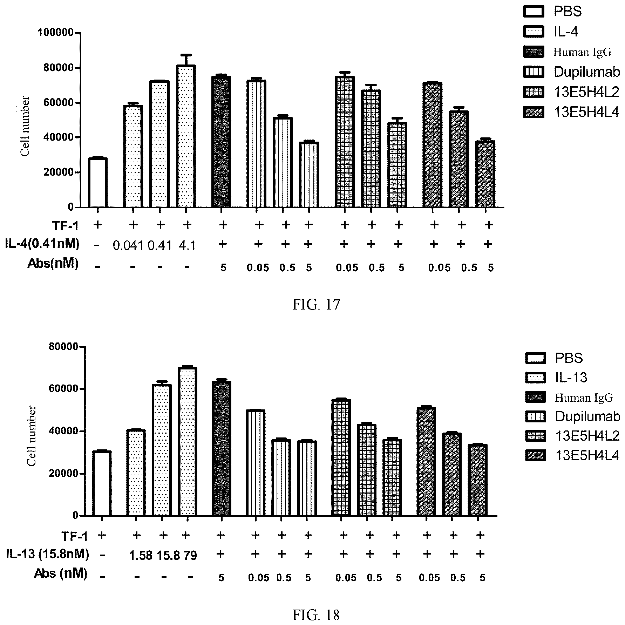

The detection results are shown in FIGs. 15-18. As can be seen in FIGs. 15-18, both human IL-4 and human IL-13 are capable of effectively promoting TF-1 cell proliferation in a dose-dependent manner; as compared to the isotype control antibody (human IgG), dupilumab, 13E5H1L1, 13E5H2L2, 13E5H4L2, and 13E5H4L4 all can specifically inhibit TF-1 cell proliferation induced by IL-4 and IL-13 in a dose-dependent manner.

The results show that 13E5H1L1, 13E5H2L2, 13E5H4L2, and 13E5H4L4 can specifically inhibit TF-1 cell proliferation induced by human IL-4 and human IL-13. 13E5H1L1 and 13E5H4L4 demonstrate comparable activities to positive control drug dupilumab.

### Example 7. 13E5H4L4 Inhibiting CD23 Expression Up-Regulation in PBMCs

This example was intended to examine the neutralizing bioactivity of 13E5H4L4 for the CD23 expression up-regulation on human PBMC surface induced by human IL-4 and human IL-13 through flow cytometry. The procedures are as follows:
Normal human peripheral blood (with heparin) was isolated by Ficoll density gradient centrifugation to obtain human fresh PBMCs. After 3 centrifugations and washes, the cells were counted and the density was adjusted to 2.5 × 10⁶ cells/mL. The cells were seeded into low attachment 96-well plates at 200 µL of PBMCs per well (i.e., 500,000 cells/well); 25 µL of the antibodies dupilumab and 13E5H4L4 (at final concentrations of 30, 3, and 0.3 nM, respectively) were added to each well, and blank control and isotype control human IgG (at a final concentration of 30 nM) were set. The cells were incubated at room temperature for 30 min. After 30 min, 25 µL of human IL-4 (at a final concentration of 100 pM) or 25 µL of human IL-13 (at a final concentration of 300 ng/mL) was added, and the system was incubated for 2.5 days. After the 2.5 days, the PBMCs of all groups were collected and transferred into 1.5-mL EP tubes, and each tube was added with 500 µL of 1% PBSA and centrifuged at 1000× g for 5 min, followed by removal of the supernatant. 50 µL of CD23-PE antibody (50-fold diluted with 1% PBSA) was added, and the mixture was incubated on ice for 40 min. After the 40 min, 1 mL of 1% PBSA was added, and the mixture was centrifuged at 1000× g for 5 min, followed by removal of the supernatant. The cells were resuspended in 200 µL of 1% PBSA, and transferred into a flow cytometry tube for assay.

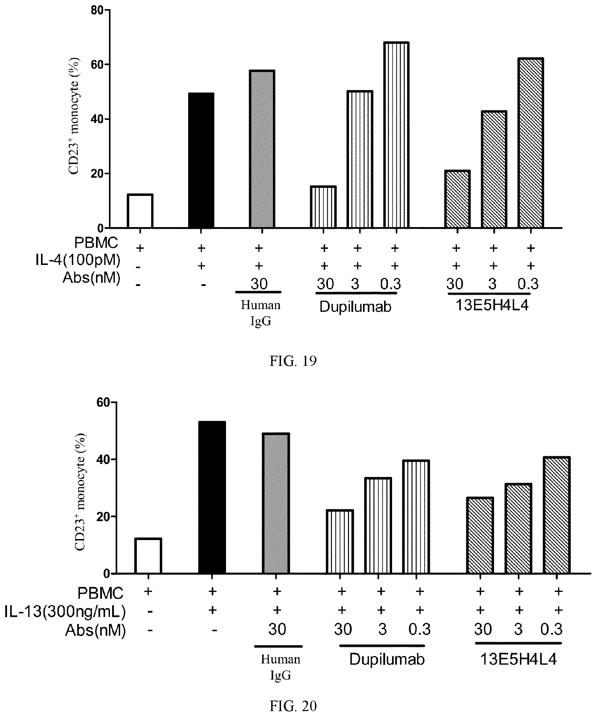

The detection results are shown in FIGs. 19 and 20.

The results show that human IL-4 and human IL-13 can up-regulate the expression level of CD23 on the surface of human PBMCs, and 13E5H4L4 can specifically bind to human IL-4RA, thereby effectively blocking the up-regulation of the CD23 expression level by IL-4 and IL-13.

### Example 8. Effect of Anti-IL-4RA Antibody on IL-4-Induced Human Eosinophil Chemotaxis

After peripheral blood erythrocytes of healthy volunteers were lysed, the cells were centrifuged at 1200 rpm for 5 min, washed with 15 mL of HBSS (i.e., Hank's balanced salt solution, constructed by Akeso Biopharma Inc.) twice, and washed with a complete medium (1640 + 10% FBS) once. The cells were resuspended in a complete medium and counted, and a granulocyte-macrophage colony stimulating factor (GM-CSF, Peprotech, Cat. No. 300-03) at a final concentration of 50 pM was added. The mixture was cultured overnight. The next day, the transwell chamber (Thermo, Cat. No. 140629) was placed in 24-well plates, 100 µL of complete medium was added to the upper chamber, and the system was equilibrated at 37 °C for 1 h. The cells were collected and separately incubated with a complete medium, 13E5H4L4 antibody (constructed by Akeso Biopharma Inc., Batch No. 201811), or isotype control antibody hIgG4 (constructed by Akeso Biopharma Inc., Ltd., Batch No. 20190910) at 37 °C for 1 h. After the 1 h, the complete medium was discarded from the upper chamber. The pre-incubation solution of the cells and the complete medium or the antibody was added to the upper chamber, and 400 µL of complete medium containing 1 nM IL-4 (Peprotech, Cat. No. 200-04) was added to the lower chamber. The chambers were incubated at 37 °C for 2.5 h. After the 2.5 h, the upper chamber was taken out, and the cells in the lower chamber were collected, washed, and centrifuged. A FITC-labeled anti-human CD66b antibody (Biolegend, Cat. No. 305104) and an APC-labeled anti-human CD16 antibody (Biolegend, Cat. No. 302012) were added, and the mixture was incubated on ice for 30 min in the dark. The cells were washed and centrifuged, and then resuspended in 200 µL of 1% PBSA(PBS + 1% BSA(Sigma, Cat. No. V900933-1KG)). The concentration of eosinophils in the lower chamber was detected on a flow cytometer. Chemotaxis inhibition rate (%) = (eosinophil concentration in the control group - eosinophil concentration in the experimental group)/eosinophil concentration in the control group × 100%.

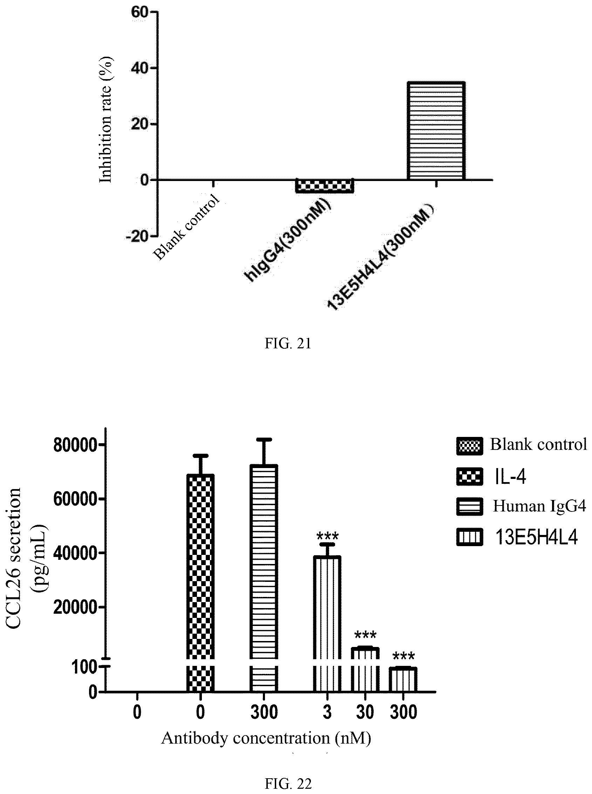

The results, as shown in FIG. 21, show that as compared to the isotype control, the anti-IL4-RA antibody 13E5H4L4 is capable of effectively inhibiting the chemotaxis of GM-CSF-treated eosinophils towards IL-4.

### Example 9. Inhibition of IL-4 Stimulation-Induced CCL26 Secretion by Anti-IL-4RA Antibody

Cytokines (IL-1β, TNF-α, IL-4, IL-13, etc.) can stimulate endothelial cells to produce a large number of chemokines. IL-4 and IL-13 over-expressed in EoE stimulate esophageal squamous cells to produce chemokine eotaxin-3 (CCL26), a potent eosinophil chemoattractant that can attract activated eosinophils to the esophagus, where the eosinophils degranulate and release toxic products leading to esophageal injury and remodeling. CCL26 has also been shown to be highly expressed in eosinophilic esophagitis lesions (Rothenberg M E. Biology and Treatment of Eosinophilic Esophagitis [J]. Gastroenterology, 2009, 137(4):1238-1249).

Human non-small cell lung cancer cell line alveolar basal epithelial cells A549 (purchased from Chinese Academy of Sciences) were collected and resuspended in DMEM with 10% FBS. The cells were seeded in 24-well plates at 5 × 10⁵ cells/well/500 µL and cultured in a 5% CO₂ incubator at 37 °C. Human umbilical vein endothelial cells HUVEC (purchased from Allcells) were collected and resuspended in an endothelial cell medium. The cells were seeded in 24-well plates at 5 × 10⁵ cells/well/500 µL and cultured in a 5% CO₂ incubator at 37 °C. After incubation for 2 h, IL-4 (purchased from Peprotech, Cat. No. 200-04) and the antibody were added for stimulation for 3 days after the cells had adhered to the wall. The culture supernatant was collected, and the CCL26 content was detected using a kit (R&D, Cat. No. DCC260B).

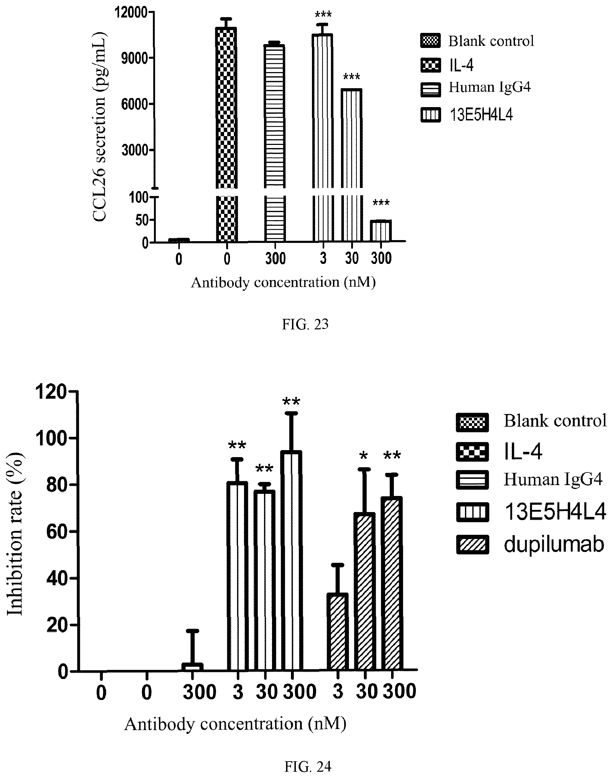

The results are shown in FIGs. 22 and 23. The results show that the anti-IL-4RA antibody 13E5H4L4 can reduce the secretion of CCL26 induced by IL-4 stimulation in both HUVEC and A549 cell systems, and has good concentration dependence. Compared to the IL-4 group, ***p < 0.001.

### Example 10 Inhibition of IL-4 Stimulation-Induced Eosinophil Chemotaxis by Anti-IL-4RA Antibody

HUVEC cells were collected and resuspended in DMEM with 10% FBS or an endothelial cell medium, and the cells were seeded in different 24-well plates at 5 × 10⁵ cells/well and cultured in a 5% CO₂ incubator at 37 °C for 2 h. After the cells had adhered to the wall, IL-4 and the antibody were added for treatment for 3 days. The culture supernatant was collected as a conditioned medium. Fresh blood (from a healthy donor) was collected with a sodium heparin anticoagulant and mixed uniformly with HBSS at 1:3. Ficoll was added to a 50 mL centrifuge tube, and the diluted blood was slowly added to the upper layer of the liquid level at a 3:4 ratio. The tube was centrifuged at 25 °C and 420× g for 30 min with an acceleration gear at 5 and a deceleration gear at 0. The upper layer was removed by pipetting, and the lower erythrocyte layer was taken and added with 1× ACK lysing buffer at a volume ratio of 1:5. The mixture was gently whirled, lysed for 15-20 min, and then centrifuged at 25 °C and 1200× g for 5 min. The granulocytes were collected, resuspended in 10 mL of HBSS, centrifuged at 25 °C and 1200× g for 5 min, and washed twice. The cells were washed once with a 1640 medium and counted with AOPI, and the cell viability was determined. Eosinophils (EOS) were isolated using an eosinophil isolation kit (Miltenyi, Cat. No. 130-092-010). The transwell chamber was placed on 24-well plates, 100 µL of 1640 medium was added to the upper chamber, and the system was equilibrated at 37 °C for 1 h. The eosinophils (EOS) were taken and added to the upper chamber at 100 µL/well (200,000 cells), and 400 µL of conditioned medium (i.e., the aforementioned culture supernatant) was added to the lower chamber. The cells were incubated in an incubator for chemotaxis for 2.5 h. The upper chamber was taken out, and the cells in the lower chamber were collected and centrifuged at 250× g for 5 min, followed by removal of the supernatant. FITC anti-human CD66b antibody (1 µL/100 µL, Biolegend, Cat. No. 305104) and PE anti-human CD16 antibody (1 µL/100 µL, Biolegend, Cat. No. 360704) were added, and the mixture was incubated. The cells were washed and then resuspended in 200 µL of 1% PBSA, and 50 µL of the cells was taken and analyzed for EOS cell count on a flow cytometer. Inhibition rate (%) = (IL-4 group - antibody group)/(IL-4 group) × 100%, and the inhibition rate of each treatment group was calculated after subtracting that of the blank control group. The results are shown in FIG. 24. The results show that in the supernatant of HUVEC cells treated with IL-4, the inhibiting effect on the chemotaxis of EOS is enhanced along with the increase of the concentration of the anti-IL-4RA antibody, which indicates that the anti-IL-4RA antibody can inhibit the chemotaxis of eosinophils induced by IL-4 stimulation in a gradient manner. In addition, in the HUVEC cell system, the chemotaxis inhibition rate of 13E5H4L4 on EOS is higher than that of the positive control dupilumab (purchased from Regeneron, Batch No. 7L615F), indicating that 13E5H4L4 has a better inhibitory effect on the chemotaxis of EOS induced by IL-4 stimulation. Compared to the IL-4 group, *p < 0.05; **p < 0.01; ***p < 0.001.

The preferred embodiments of the present invention have been described above in detail, but the present invention is not limited to the embodiments. Those skilled in the art can make various equivalent modifications or replacements without violating the spirit of the present invention. These equivalent modifications or replacements are included in the scope defined by the claims of the present application.

### Sequences (Note: those underlined are CDR regions)

**13E5 heavy chain variable region:** 
GAGGTGCAGCTGCAGCAGAGCGGACCAGAGCTGGTGAAGCCTGGC
GCCTCTGTGAAGATCAGCTGTAAGACCTCCGGCTACACCTTCACAGA
GTATACAATCCACTGGGTGAAGCAGAACCACGGCAAGAGCCTGGAG
TGGATCGGCGGCATCAATCCTAACAATGGCGGCACCGTGTACAACCA
GAACTTCAAGGGCAAGGCCACCCTGACAGTGGACAAGAGCTCCTCT
ACCGCCTATATGGAGCTGAGGTCTCTGACAAGCGAGGACTCCGCCGT
GTACTATTGCGCCAGAGTGCGGAGAGGCATGGATTACTGGGGCCAG
GGCACCTCCGTGACAGTGAGCTCC (SEQ ID NO: 1)
 
EVQLQQSGPELVKPGASVKISCKTSGYTFTEYTIHWVKQNHGKSLEWI
GGINPNNGGTVYNQNFKGKATLTVDKSSSTAYMELRSLTSEDSAVYYC
ARVRRGMDYWGQGTSVTVSS (SEQ ID NO: 2)
**13E5 light chain variable region** 
GACATCGTGATGACCCAGTCCCACAAGTTTATGTCCACATCTGTGGG
CGACAGGGTGTCCATCACCTGTAAGGCCTCTCAGGATGTGACCACA
 
GCCGTGGCCTGGTACCAGCAGAAGCCAGGCCAGTCTCCCAAGCTGC
TGATCTATAGCGCCTCCTACCGGTATACAGGCGTGCCCGACAGATTCA
CCGGCTCTGGCAGCGGCACAGATTTCACCTTTACAATCAGCTCCGTG
CAGGCAGAGGACCTGGCCGTGTACTATTGCCAGCAGCACTACTCTGC
CCCTTGGACCTTCGGCGGAGGAACAAACCTGGAGATCAAG (SEQ ID
NO: 3)
 
DIVMTQSHKFMSTSVGDRVSITCKASQDVTTAVAWYQQKPGQSPKLLI
YSASYRYTGVPDRFTGSGSGTDFTFTISSVQAEDLAVYYCQQHYSAPW
TFGGGTNLEIK (SEQ ID NO: 4)
**13E5 CDR**
   HCDR1: GYTFTEYT (SEQ ID NO: 5)
   HCDR2: INPNNGGT (SEQ ID NO: 6)
   HCDR3: ARVRRGMDY (SEQ ID NO: 7)
   LCDR1: QDVTTA (SEQ ID NO: 8)
   LCDR2: SAS (SEQ ID NO: 9)
   LCDR3: QQHYSAPWT (SEQ ID NO: 10)
**13E5H1 heavy chain variable region** 
CAGGTGCAGCTGCAGCAGTCCGGAGCAGAGGTGGTGAAGCCAGGA
GCCAGCGTGAAGATCTCCTGTAAGACCTCTGGCTACACCTTCACAGA
GTATACAATCCACTGGGTGAAGCAGGCACACGGACAGAGCCTGGAG
TGGATCGGCGGCATCAACCCTAACAATGGCGGCACCGTGTACAATCA
GAAGTTTCAGGGCAAGGCCACCCTGACAGTGGACAAGTCTACCAGC
ACAGCCTATATGGAGCTGAGGTCCCTGACCTCTGAGGACACAGCCGT
GTACTATTGCGCCCGGGTGCGGAGAGGCATGGATTACTGGGGCCAGG
GCACCTCCGTGACAGTGAGCTCC (SEQ ID NO: 11)
 
QVQLQQSGAEVVKPGASVKISCKTSGYTFTEYTIHWVKQAHGQSLEWI
GGINPNNGGTVYNQKFQGKATLTVDKSTSTAYMELRSLTSEDTAVYYC
ARVRRGMDYWGQGTSVTVSS (SEQ ID NO: 12)
**13E5L1 light chain variable region** 
GACATCCAGATGACCCAGTCTCCTAAGTCTCTGAGCACATCCGTGGG
CGACCGGGTGACCATCACATGTAGAGCCAGCCAGGATGTGACCACA
GCAGTGGCATGGTACCAGCAGAAGCCTGGCAAGTCCCCTAAGCTGC
TGATCTATTCTGCCAGCTACAGGTATACCGGAGTGCCATCTCGGTTCT
CCGGCTCTGGCAGCGGCACAGACTTCACCTTTACAATCAGCTCCGTG
CAGCCAGAGGATCTGGCCACCTACTATTGCCAGCAGCACTACAGCGC
CCCATGGACCTTTGGCGGAGGAACAAACCTGGAGATCAAG (SEQ ID
NO: 13)
 
DIQMTQSPKSLSTSVGDRVTITCRASQDVTTAVAWYQQKPGKSPKLLIY
SASYRYTGVPSRFSGSGSGTDFTFTISSVQPEDLATYYCQQHYSAPWTF
GGGTNLEIK (SEQ ID NO: 14)
**13E5H2 heavy chain variable region** 
CAGGTGCAGCTGGTGCAGTCCGGAGCAGAGGTGGTGAAGCCAGGA
GCCAGCGTGAAGGTGTCCTGTAAGACCTCTGGCTACACCTTCACAG
AGTATACAATCCACTGGGTGCGGCAGGCACCAGGACAGTCTCTGGA
GTGGATCGGCGGCATCAACCCTAACAATGGCGGCACCAACTACAATC
AGAAGTTTCAGGGCAAGGTGACCCTGACAGTGGACAAGTCCACCTC
TACAGCCTATATGGAGCTGAGCTCCCTGAGGTCTGAGGACACAGCCG
TGTACTATTGCGCCCGCGTGCGGAGAGGCATGGATTACTGGGGCCAG
GGCACCAGCGTGACAGTGTCTAGC (SEQ ID NO: 15)
 
QVQLVQSGAEVVKPGASVKVSCKTSGYTFTEYTIHWVRQAPGQSLEWI
GGINPNNGGTNYNQKFQGKVTLTVDKSTSTAYMELSSLRSEDTAVYYC
ARVRRGMDYWGQGTSVTVSS (SEQ ID NO: 16)
**13E5L2 light chain variable region** 
GACATCCAGATGACCCAGAGCCCTAGCTCCCTGAGCGCCTCCGTGG
GCGACAGGGTGACCATCACATGTAGAGCCTCCCAGGATGTGACCAC
AGCAGTGGCATGGTACCAGCAGAAGCCAGGCAAGGCCCCTAAGCTG
CTGATCTACTCCGCCTCTAGCAGGTATACCGGAGTGCCATCTCGGTTC
TCTGGCAGCGGCTCCGGCACAGACTTTACCCTGACAATCTCCTCTGT
GCAGCCAGAGGATCTGGCCACATACTATTGCCAGCAGCACTATTCTG
CCCCCTGGACCTTTGGCGGCGGCACAAACCTGGAGATCAAG (SEQ ID
NO: 17)
 
DIQMTQSPSSLSASVGDRVTITCRASQDVTTAVAWYQQKPGKAPKLLIY
SASSRYTGVPSRFSGSGSGTDFTLTISSVQPEDLATYYCQQHYSAPWTF
GGGTNLEIK (SEQ ID NO: 18)
**13E5H3 heavy chain variable region** 
CAGGTGCAGCTGGTGCAGTCCGGAGCAGAGGTGGTGAAGCCAGGA
GCCAGCGTGAAGGTGTCCTGTAAGGCCTCTGGCTACACCTTCACAG
AGTATACCATCCACTGGGTGCGGCAGGCACCAGGACAGGGACTGGA
GTGGATCGGCGGCATCAACCCTAACAATGGCGGCACAAATTACGCCC
AGAAGTTTCAGGGCAGGGTGACCATCACAGTGGACAAGTCCACCTC
TACAGCCTATATGGAGCTGAGCTCCCTGAGGTCTGAGGACACCGCCG
TGTACTATTGCGCCCGCGTGCGGAGAGGCATGGATTACTGGGGCCAG
GGCACCAGCGTGACAGTGTCTAGC (SEQ ID NO: 19)
 
QVQLVQSGAEVVKPGASVKVSCKASGYTFTEYTIHWVRQAPGQGLEW
IGGINPNNGGTNYAQKFQGRVTITVDKSTSTAYMELSSLRSEDTAVYYC
ARVRRGMDYWGQGTSVTVSS (SEQ ID NO: 20)
**13E5L3 light chain variable region** 
GACATCCAGATGACCCAGAGCCCTAGCTCCCTGAGCGCCTCCGTGG
GCGACAGGGTGACCATCACATGTAGAGCCTCCCAGGATGTGACCAC
AGCCCTGGCATGGTACCAGCAGAAGCCAGGCAAGGCCCCTAAGCTG
CTGATCTACTCCGCCTCTAGCCTGTATACCGGAGTGCCATCTCGGTTC
TCTGGCAGCGGCTCCGGCACAGACTTTACCCTGACAATCTCCTCTCT
GCAGCCAGAGGATTTCGCCACATACTATTGCCAGCAGCACTATTCTG
CCCCCTGGACCTTTGGCGGCGGCACAAACCTGGAGATCAAG (SEQ ID
NO: 21)
 
DIQMTQSPSSLSASVGDRVTITCRASQDVTTALAWYQQKPGKAPKLLIY
SASSLYTGVPSRFSGSGSGTDFTLTISSLQPEDFATYYCQQHYSAPWTFG
GGTNLEIK (SEQ ID NO: 22)
**13E5H4 heavy chain variable region** 
CAGGTGCAGCTGGTGCAGTCCGGAGCAGAGGTGGTGAAGCCAGGA
GCCAGCGTGAAGGTGTCCTGTAAGACCTCTGGCTACACCTTCACAG
AGTATACAATCCACTGGGTGCGGCAGGCACCAGGACAGTCTCTGGA
GTGGATCGGCGGCATCAACCCTAACAATGGCGGCACCgtcTACAATCA
GAAGTTTCAGGGCAAGGTGACCCTGACAGTGGACAAGTCCACCTCT
ACAGCCTATATGGAGCTGAGCTCCCTGAGGTCTGAGGACACAGCCGT
GTACTATTGCGCCCGCGTGCGGAGAGGCATGGATTACTGGGGCCAGG
GCACCAGCGTGACAGTGTCTAGC (SEQ ID NO: 23)
 
QVQLVQSGAEVVKPGASVKVSCKTSGYTFTEYTIHWVRQAPGQSLEWI
GGINPNNGGTVYNQKFQGKVTLTVDKSTSTAYMELSSLRSEDTAVYYC
ARVRRGMDYWGQGTSVTVSS (SEQ ID NO: 24)
**13E5L4: light chain variable region** 
GACATCCAGATGACCCAGAGCCCTAGCTCCCTGAGCGCCTCCGTGG
GCGACAGGGTGACCATCACATGTAGAGCCTCCCAGGATGTGACCAC
AGCAGTGGCATGGTACCAGCAGAAGCCAGGCAAGGCCCCTAAGCTG
CTGATCTACTCCGCCTCTTAcAGGTATACCGGAGTGCCATCTCGGTTC
TCTGGCAGCGGCTCCGGCACAGACTTTACCCTGACAATCTCCTCTGT
GCAGCCAGAGGATCTGGCCACATACTATTGCCAGCAGCACTATTCTG
CCCCCTGGACCTTTGGCGGCGGCACAAACCTGGAGATCAAG (SEQ ID
NO: 25)
 
DIQMTQSPSSLSASVGDRVTITCRASQDVTTAVAWYQQKPGKAPKLLIY
SASYRYTGVPSRFSGSGSGTDFTLTISSVQPEDLATYYCQQHYSAPWTF
GGGTNLEIK (SEQ ID NO: 26)
**13E5 heavy chain framework region**
   FR-H1: EVQLQQSGPELVKPGASVKISCKTS (SEQ ID NO: 27)
   FR-H2: IHWVKQNHGKSLEWIGG (SEQ ID NO: 28)
   FR-H3: VYNQNFKGKATLTVDKSSSTAYMELRSLTSEDSAVYYC (SEQ ID NO: 29)
   FR-H4: WGQGTSVTVSS (SEQ ID NO: 30)
   :
**13E5 light chain framework region**
   FR-L1: DIVMTQSHKFMSTSVGDRVSITCKAS (SEQ ID NO: 31)
   FR-L2: VAWYQQKPGQSPKLLIY (SEQ ID NO: 32)
   FR-L3: YRYTGVPDRFTGSGSGTDFTFTISSVQAEDLAVYYC (SEQ ID NO: 33)
   FR-L4: FGGGTNLEIK (SEQ ID NO: 34)
**13E5H1 framework region:**
   FR-H1: QVQLQQSGAEVVKPGASVKISCKTS (SEQ ID NO: 35)
   FR-H2: IHWVKQAHGQSLEWIGG (SEQ ID NO: 36)
   FR-H3: VYNQKFQGKATLTVDKSTSTAYMELRSLTSEDTAVYYC (SEQ ID NO: 37)
   FR-H4: WGQGTSVTVSS (SEQ ID NO: 38)
**13E5L1 framework region:**
   FR-L1: DIQMTQSPKSLSTSVGDRVTITCRAS (SEQ ID NO: 39)
   FR-L2: VAWYQQKPGKSPKLLIY (SEQ ID NO: 40)
   FR-L3: YRYTGVPSRFSGSGSGTDFTFTISSVQPEDLATYYC (SEQ ID NO: 41)
   FR-L4: FGGGTNLEIK (SEQ ID NO: 42)
**13E5H2 framework region:**
   FR-H1: QVQLVQSGAEVVKPGASVKVSCKTS (SEQ ID NO: 43)
   FR-H2: IHWVRQAPGQSLEWIGG (SEQ ID NO: 44)
   FR-H3: NYNQKFQGKVTLTVDKSTSTAYMELSSLRSEDTAVYYC (SEQ ID NO: 45)
   FR-H4: WGQGTSVTVSS (SEQ ID NO: 46)
**13E5L2 framework region:**
   FR-L1: DIQMTQSPSSLSASVGDRVTITCRAS (SEQ ID NO: 47)
   FR-L2: VAWYQQKPGKAPKLLIY (SEQ ID NO: 48)
   FR-L3: SRYTGVPSRFSGSGSGTDFTLTISSVQPEDLATYYC (SEQ ID NO: 49)
   FR-L4: FGGGTNLEIK (SEQ ID NO: 50)
**13E5H3 framework region:**
   FR-H1: QVQLVQSGAEVVKPGASVKVSCKAS (SEQ ID NO: 51)
   FR-H2: IHWVRQAPGQGLEWIGG (SEQ ID NO: 52)
   FR-H3: NYAQKFQGRVTITVDKSTSTAYMELSSLRSEDTAVYYC (SEQ ID NO: 53)
   FR-H4: WGQGTSVTVSS (SEQ ID NO: 54)
**13E5L3 framework region:**
   FR-L1: DIQMTQSPSSLSASVGDRVTITCRAS (SEQ ID NO: 55)
   FR-L2: LAWYQQKPGKAPKLLIY (SEQ ID NO: 56)
   FR-L3: SLYTGVPSRFSGSGSGTDFTLTISSLQPEDFATYYC (SEQ ID NO: 57)
   FR-L4: FGGGTNLEIK (SEQ ID NO: 58)
**13E5H4: framework region:**
   FR-H1: QVQLVQSGAEVVKPGASVKVSCKTS (SEQ ID NO: 59)
   FR-H2: IHWVRQAPGQSLEWIGG (SEQ ID NO: 60)
   FR-H3: VYNQKFQGKVTLTVDKSTSTAYMELSSLRSEDTAVYYC (SEQ ID NO: 61)
   FR-H4: WGQGTSVTVSS (SEQ ID NO: 62)
**13E5L4 framework region:**
   FR-L1: DIQMTQSPSSLSASVGDRVTITCRAS (SEQ ID NO: 63)
   FR-L2: VAWYQQKPGKAPKLLIY (SEQ ID NO: 64)
   FR-L3: YRYTGVPSRFSGSGSGTDFTLTISSVQPEDLATYYC (SEQ ID NO: 65)
   FR-L4: FGGGTNLEIK (SEQ ID NO: 66)
**Sequence of mFc tag:** 
PRGPTIKPCPPCKCPAPNLLGGPSVFIFPPKIKDVLMISLSPIVTCVVVDV
SEDDPDVQISWFVNNVEVHTAQTQTHREDYNSTLRVVSALPIQHQDW
MSGKEFKCKVNNKDLPAPIERTISKPKGSVRAPQVYVLPPPEEEMTKKQ
VTLTCMVTDFMPEDIYVEWTNNGKTELNYKNTEPVLDSDGSYFMYSK
LRVEKKNWVERNSYSCSVVHEGLHNHHTTKSFSRTPGK (SEQ ID NO:
67)
**hIgG heavy chain sequence** 
EVQLEQSGAELMKPGASVKISCKATGYTFTTYWIEWIKQRPGHSLEWI
GEILPGSDSTYYNEKVKGKVTFTADASSNTAYMQLSSLTSEDSAVYYCA
RGDGFYVYWGQGTTLTVSSASTKGPSVFPLAPCSRSTSESTAALGCLVK
DYFPEPVTVSWNSGALTSGVHTFPAVLQSSGLYSLSSVVTVPSSSLGTKT
YTCNVDHKPSNTKVDKRVESKYGPPCPPCPAPEFLGGPSVFLFPPKPKD
TLMISRTPEVTCVVVDVSQEDPEVQFNWYVDGVEVHNAKTKPREEQF
NSTYRVVSVLTVLHQDWLNGKEYKCKVSNKGLPSSIEKTISKAKGQPR
EPQVYTLPPSQEEMTKNQVSLTCLVKGFYPSDIAVEWESNGQPENNYK
TTPPVLDSDGSFFLYSRLTVDKSRWQEGNVFSCSVMHEALHNHYTQKS
LSLSLGK (SEQ ID NO: 68)
**hIgG light chain sequence** 
DIELTQSPATLSVTPGDSVSLSCRASQSISNNLHWYQQKSHESPRLLIKY
TSQSMSGIPSRFSGSGSGTDFTLSINSVETEDFGVYFCQQSGSWPRTFGG
GTKLDIKRTVAAPSVFIFPPSDEQLKSGTASVVCLLNNFYPREAKVQWK
 
VDNALQSGNSQESVTEQDSKDSTYSLSSTLTLSKADYEKHKVYACEVT
HQGLSSPVTKSFNRGEC (SEQ ID NO: 69)
**VAB 16F3-1 VH-2** 
EVQLVESGGGLVQPGRSLRLSCEASGFTFDDYAMHWVRQAPGKGLEW
VSGLSRTSVSIGYADSVKGRFTISRDNAKNSLYLEMNSLRPEDTALYYC
AKWGTRGYFDYWGQGTLVTVSS (SEQ ID NO: 70)
**16F3-1 VL**

DIQMTQSPSSVSASVGDRVTITCRASQDISIWLAWYQQSPGKAPKLLIN
VASRLQSGVPSRFSGSGSGTDFTLTINSLQPEDFVTYYCQQANSFPITFG
QGTRLATK (SEQ ID NO: 71)

## Claims

1. An antibody or an antigen-binding fragment thereof, particularly, the antibody or the antigen-binding fragment thereof binding to IL-4RA, preferably human IL-4RA, for use in treating eosinophilic esophagitis, wherein: according to the Kabat, IMGT, Chothia, or AbM numbering system, the antibody comprises:
an HCDR1, an HCDR2, and an HCDR3 comprised in a heavy chain variable region set forth in SEQ ID NO: 2, and an LCDR1, an LCDR2, and an LCDR3 comprised in a light chain variable region set forth in SEQ ID NO: 4;
preferably, according to the IMGT numbering system, the antibody comprises:
an HCDR1 comprising or consisting of a sequence set forth in SEQ ID NO: 5 or a variant thereof,
an HCDR2 comprising or consisting of a sequence set forth in SEQ ID NO: 6 or a variant thereof, and
an HCDR3 comprising or consisting of a sequence set forth in SEQ ID NO: 7 or a variant thereof; and
the antibody further comprises:
an LCDR1 comprising or consisting of a sequence set forth in SEQ ID NO: 8 or a variant thereof,
an LCDR2 comprising or consisting of a sequence set forth in SEQ ID NO: 9 or a variant thereof, and
an LCDR3 comprising or consisting of a sequence set forth in SEQ ID NO: 10 or a variant thereof,
wherein a sequence of the variant is a sequence having at least 90%, preferably at least 91%, 92%, 93%, 94%, 95%, 96%, 97%, 98%, or 99% sequence identity to the corresponding sequence.

2. The antibody according to claim 1, wherein the antibody comprises:
(1)
(i) a heavy chain variable region comprising or consisting of:
an amino acid sequence set forth in SEQ ID NO: 2 or a variant thereof, and
(ii) a light chain variable region comprising or consisting of:
an amino acid sequence set forth in SEQ ID NO: 4 or a variant thereof;
(2)
(i) a heavy chain variable region comprising or consisting of:
an amino acid sequence set forth in SEQ ID NO: 12 or a variant thereof, and
(ii) a light chain variable region comprising or consisting of:
an amino acid sequence set forth in SEQ ID NO: 14 or a variant thereof;
(3)
(i) a heavy chain variable region comprising or consisting of:
an amino acid sequence set forth in SEQ ID NO: 16 or a variant thereof, and
(ii) a light chain variable region comprising or consisting of:
an amino acid sequence set forth in SEQ ID NO: 18 or a variant thereof;
(4)
(i) a heavy chain variable region comprising or consisting of:
an amino acid sequence set forth in SEQ ID NO: 20 or a variant thereof, and
(ii) a light chain variable region comprising or consisting of:
an amino acid sequence set forth in SEQ ID NO: 22 or a variant thereof;
(5)
(i) a heavy chain variable region comprising or consisting of:
an amino acid sequence set forth in SEQ ID NO: 24 or a variant thereof, and
(ii) a light chain variable region comprising or consisting of:
an amino acid sequence set forth in SEQ ID NO: 26 or a variant thereof;
(6)
(i) a heavy chain variable region comprising or consisting of:
an amino acid sequence set forth in SEQ ID NO: 24 or a variant thereof, and
(ii) a light chain variable region comprising or consisting of:
an amino acid sequence set forth in SEQ ID NO: 18 or a variant thereof,
alternatively wherein a sequence of the variant is a sequence having at least 80%, 85%, or 90%, preferably at least 91%, 92%, 93%, 94%, 95%, 96%, 97%, 98%, or 99% sequence identity to the corresponding sequence.

3. The antibody or the antigen-binding fragment thereof according to any one of claims 1 and 2, wherein the antibody further comprises framework regions FR-H1, FR-H2, FR-H3, and FR-H4 in the heavy chain variable region, and framework regions FR-L1, FR-L2, FR-L3, and FR-L4 in the light chain variable region, wherein
(1) the FR-H1 comprises or consists of the amino acid sequence of SEQ ID NO: 27 or a variant thereof;
the FR-H2 comprises or consists of the amino acid sequence of SEQ ID NO: 28 or a variant thereof;
the FR-H3 comprises or consists of the amino acid sequence of SEQ ID NO: 29 or a variant thereof;
the FR-H4 comprises or consists of the amino acid sequence of SEQ ID NO: 30 or a variant thereof;
the FR-L1 comprises or consists of the amino acid sequence of SEQ ID NO: 31 or a variant thereof;
the FR-L2 comprises or consists of the amino acid sequence of SEQ ID NO: 32 or a variant thereof;
the FR-L3 comprises or consists of the amino acid sequence of SEQ ID NO: 33 or a variant thereof; and
the FR-L4 comprises or consists of the amino acid sequence of SEQ ID NO: 34 or a variant thereof;
(2) the FR-H1 comprises or consists of the amino acid sequence of SEQ ID NO: 35 or a variant thereof;
the FR-H2 comprises or consists of the amino acid sequence of SEQ ID NO: 36 or a variant thereof;
the FR-H3 comprises or consists of the amino acid sequence of SEQ ID NO: 37 or a variant thereof;
the FR-H4 comprises or consists of the amino acid sequence of SEQ ID NO: 38 or a variant thereof;
the FR-L1 comprises or consists of the amino acid sequence of SEQ ID NO: 39 or a variant thereof;
the FR-L2 comprises or consists of the amino acid sequence of SEQ ID NO: 40 or a variant thereof;
the FR-L3 comprises or consists of the amino acid sequence of SEQ ID NO: 41 or a variant thereof; and
the FR-L4 comprises or consists of the amino acid sequence of SEQ ID NO: 42 or a variant thereof;
(3) the FR-H1 comprises or consists of the amino acid sequence of SEQ ID NO: 43 or a variant thereof;
the FR-H2 comprises or consists of the amino acid sequence of SEQ ID NO: 44 or a variant thereof;
the FR-H3 comprises or consists of the amino acid sequence of SEQ ID NO: 45 or a variant thereof;
the FR-H4 comprises or consists of the amino acid sequence of SEQ ID NO: 46 or a variant thereof;
the FR-L1 comprises or consists of the amino acid sequence of SEQ ID NO: 47 or a variant thereof;
the FR-L2 comprises or consists of the amino acid sequence of SEQ ID NO: 48 or a variant thereof;
the FR-L3 comprises or consists of the amino acid sequence of SEQ ID NO: 49 or a variant thereof; and
the FR-L4 comprises or consists of the amino acid sequence of SEQ ID NO: 50 or a variant thereof;
(4) the FR-H1 comprises or consists of the amino acid sequence of SEQ ID NO: 51 or a variant thereof;
the FR-H2 comprises or consists of the amino acid sequence of SEQ ID NO: 52 or a variant thereof;
the FR-H3 comprises or consists of the amino acid sequence of SEQ ID NO: 53 or a variant thereof;
the FR-H4 comprises or consists of the amino acid sequence of SEQ ID NO: 54 or a variant thereof;
the FR-L1 comprises or consists of the amino acid sequence of SEQ ID NO: 55 or a variant thereof;
the FR-L2 comprises or consists of the amino acid sequence of SEQ ID NO: 56 or a variant thereof;
the FR-L3 comprises or consists of the amino acid sequence of SEQ ID NO: 57 or a variant thereof; and
the FR-L4 comprises or consists of the amino acid sequence of SEQ ID NO: 58 or a variant thereof;
(5) the FR-H1 comprises or consists of the amino acid sequence of SEQ ID NO: 59 or a variant thereof;
the FR-H2 comprises or consists of the amino acid sequence of SEQ ID NO: 60 or a variant thereof;
the FR-H3 comprises or consists of the amino acid sequence of SEQ ID NO: 61 or a variant thereof;
the FR-H4 comprises or consists of the amino acid sequence of SEQ ID NO: 62 or a variant thereof;
the FR-L1 comprises or consists of the amino acid sequence of SEQ ID NO: 63 or a variant thereof;
the FR-L2 comprises or consists of the amino acid sequence of SEQ ID NO: 64 or a variant thereof;
the FR-L3 comprises or consists of the amino acid sequence of SEQ ID NO: 65 or a variant thereof; and
the FR-L4 comprises or consists of the amino acid sequence of SEQ ID NO: 66 or a variant thereof,
wherein a sequence of the variant has at least 80%, 85%, or 90%, preferably at least 91%, 92%, 93%, 94%, 95%, 96%, 97%, 98%, or 99% sequence identity to the corresponding sequence.

4. The antibody or the antigen-binding fragment thereof according to any one of claims 1-3, wherein the antibody is a humanized antibody, a chimeric antibody, or a multispecific antibody (e.g., bispecific antibody).

5. The antibody or the antigen-binding fragment thereof according to claim 4, wherein the constant region of the antibody is humanized, preferably derived from human IgG, more preferably IgG4.

6. The antibody or the antigen-binding fragment thereof according to claim 5, wherein the heavy chain constant region of the antibody is an Ig gamma-4 chain C region, more preferably the Ig gamma-4 chain C region of GenBank ACCESSION No: P01861.1; the light chain constant region is an Ig kappa chain C region, more preferably the Ig kappa chain C region of GenBank ACCESSION No: P01834.

7. The antibody or the antigen-binding fragment thereof according to any one of claims 1-6, wherein the antigen-binding fragment is selected from Fab, Fab', F(ab')₂, Fd, Fv, dAb, Fab/c, a complementarity determining region (CDR) fragment, a single chain antibody (e.g., scFv), a bivalent antibody, or a domain antibody.

8. A biomaterial, wherein the biomaterial is selected from an antibody conjugate, a multispecific antibody (preferably a bispecific antibody), a fusion protein, or a pharmaceutical composition for use in treating eosinophilic esophagitis, and comprises the antibody or the antigen-binding fragment thereof according to any one of claims 1-7,
wherein preferably, the antibody conjugate further comprises a conjugated moiety coupled to the antibody or the antigen-binding fragment thereof, the conjugated moiety being selected from a purification tag (e.g., a His tag), a cytotoxic agent, a detectable label, a radioisotope, a luminescent substance, a colored substance, an enzyme, or polyethylene glycol;
preferably, the multispecific antibody further comprises an antibody or an antigen-binding fragment against another antigen and/or another antigenic epitope;
preferably, the pharmaceutical composition further comprises a pharmaceutically acceptable carrier and/or excipient;
preferably, the pharmaceutical composition is used alone or in combination with one or more medicaments;
preferably, the pharmaceutical composition is in a dosage form suitable for oral administration to the gastrointestinal (GI) tract, preferably the dosage form being selected from tablets, capsules, pills, powders, granules, emulsions, microemulsions, solutions, suspensions, syrups, and elixirs; or the pharmaceutical composition is in a dosage form suitable for subcutaneous injection, intradermal injection, intravenous injection, intramuscular injection, or intralesional injection.

9. Use of the antibody or the antigen-binding fragment thereof according to any one of claims 1-7, and the biomaterial according to claim 8 in preparing a medicament or kit for treating eosinophilic esophagitis, wherein preferably, the kit further comprises an additional therapeutic agent selected from one or more of the following: an IL-4/IL-13 pathway inhibitor, an IL-1β inhibitor, an IL-5 inhibitor, an IL-8 inhibitor, an IL-9 inhibitor, an IL-13 inhibitor, an IL-17 inhibitor, an IL-25 inhibitor, a JAK inhibitor, a STAT6 inhibitor, a TNF inhibitor, an eotaxin-3 (CCL26) inhibitor, an IgE inhibitor, a prostaglandin D2 inhibitor, an immunosuppressive agent, a corticosteroid, a glucocorticoid, a long-acting β2-agonist (e.g., salmeterol or formoterol), a proton pump inhibitor, a decongestant, an antihistamine and a non-steroidal anti-inflammatory agent (NSAID), an NSAID (non-steroidal anti-inflammatory drug), an antibiotic, an antibacterial agent, an antiviral agent, an antifungal agent, and a drug for treating tumors (preferably a chemotherapeutic agent or a growth inhibitor, a targeted therapeutic agent, an antibody-drug conjugate, a T cell expressing chimeric antigen receptors, an antibody or an antigen-binding fragment thereof, an angiogenesis inhibitor, an anti-tumor agent, a cancer vaccine, an adjuvant and a combination thereof, an alkylating agent, an antimetabolite, an antibiotic, a botanical drug and/or a hormonal drug),
wherein the IL-4/IL-13 pathway inhibitor is not the antibody or the antigen-binding fragment thereof according to any one of claims 1-7;
preferably, the IL-4/IL-13 pathway inhibitor is selected from an anti-IL-4 antibody, an anti-IL-13 antibody, an anti-IL-4/IL-13 antibody, an IL-4 receptor (IL-4R) inhibitor (e.g., a dupilumab antibody or a biological equivalent thereof, AMG317, or MEDI9314), a JAK inhibitor, and a STAT6 inhibitor.

10. A method for treating eosinophilic esophagitis, comprising administering to a subject in need the antibody or the antigen-binding fragment thereof according to any of claims 1-7 and the biomaterial according to claim 8, wherein preferably, the subject is selected from a mammal (e.g., a rodent, a feline, a canine, and a primate), and preferably the subject is a human, more preferably an infant aged 0-1, a child aged 1-5, a juvenile aged 6-12, an adolescent aged 12-18, and an adult aged above 18.

11. The method according to claim 10, further comprising administering to the subject an additional therapeutic agent or a combination therapy, wherein the therapeutic agent is selected from one or more of the following: an IL-4/IL-13 pathway inhibitor, an IL-1β inhibitor, an IL-5 inhibitor, an IL-8 inhibitor, an IL-9 inhibitor, an IL-13 inhibitor, an IL-17 inhibitor, an IL-25 inhibitor, a JAK inhibitor, a STAT6 inhibitor, a TNF inhibitor, an eotaxin-3 (CCL26) inhibitor, an IgE inhibitor, a prostaglandin D2 inhibitor, an immunosuppressive agent, a corticosteroid, a glucocorticoid, a long-acting β2-agonist (e.g., salmeterol or formoterol), a proton pump inhibitor, a decongestant, an antihistamine and a non-steroidal anti-inflammatory agent (NSAID), an NSAID (non-steroidal anti-inflammatory drug), an antibiotic, an antibacterial agent, an antiviral agent, an antifungal agent, a drug for treating tumors (preferably a chemotherapeutic agent or a growth inhibitor, a targeted therapeutic agent, an antibody-drug conjugate, a T cell expressing chimeric antigen receptors, an antibody or an antigen-binding fragment thereof, an angiogenesis inhibitor, an anti-tumor agent, a cancer vaccine, an adjuvant and a combination thereof, an alkylating agent, an antimetabolite, an antibiotic, a botanical drug and/or a hormonal drug), or a combination thereof, wherein the combination therapy comprises diet therapy and bougienage of oesophagus;
wherein the IL-4/IL-13 pathway inhibitor is not the antibody or the antigen-binding fragment thereof according to any one of claims 1-7;
preferably, the IL-4/IL-13 pathway inhibitor is selected from an anti-IL-4 antibody, an anti-IL-13 antibody, an anti-IL-4/IL-13 antibody, an IL-4 receptor (IL-4R) inhibitor (e.g., a dupilumab antibody or a biological equivalent thereof, AMG317, or MEDI9314), a JAK inhibitor, and a STAT6 inhibitor;
more preferably, the additional therapeutic agent is administered simultaneously or sequentially with the antibody or the antigen-binding fragment thereof according to any one of claims 1-7.

12. A hybridoma cell line having an accession number of CCTCC NO: C2018131 or a monoclonal antibody secreted by the hybridoma cell line for use in treating eosinophilic esophagitis.
